# EUROPEAN PATENT APPLICATION

(11) **EP 4 382 137 A1**
(43) Date of publication of application: **12.06.2024**
(21) Application number: 22853494.7
(22) Date of filing: 04.08.2022
(51) Int. Cl.: A61K 51/12

(54) **NOVEL COMPOUND COMPRISING ESTER GROUP AND USES THEREOF**

(30) Priority: 04.08.2021 KR 20210102453
(71) Applicant: Kyungpook National University Industry-Academic Cooperation Foundation, Daegu 41566 (KR)
(72) Inventor: YOO, Jeongsoo, Daegu 41566 (KR); KIM, Kyungwon, Daegu 41566 (KR)
(74) Representative: Hoefer & Partner Patentanwälte mbB
(86) International application number: PCT/KR2022/011570
(87) International publication number: WO 2023/014119

(57) **Abstract**

The present invention relates to a novel compound comprising an ester group and uses thereof and, more particularly, to a novel compound that exhibits excellent visualization ability in a tumor by using the difference in activity of esterase for each organ in the body, and to uses therefor for diagnosing or treating cancer. A novel compound of chemical Formulas 1 to 8 according to the present invention exhibits selectivity according to the difference in activity of esterase for each organ due to ester residue in a structure, and thus is extremely effective in visualizing pancreatic cancer, etc.

## Description

### TECHNICAL FIELD

The present application claims priority to Korean Patent Application No. 2021-0102453 filed on August 4, 2021, and the entire specification thereof is incorporated herein by reference.

The present invention relates to a novel compound comprising an ester group and uses thereof and, more particularly, to a novel compound that exhibits excellent visualization ability in a tumor by using the difference in activity of esterase for each organ in the body, and to uses therefor for diagnosing or treating cancer.

### BACKGROUND ART

An ideal tumor contrast agent should visualize only the tumor without background noise such as surrounding tissues and organs. However, selective tumor targeting by inhibiting contrast agent uptake in other organs has not been achieved in practice. Recently, various nanoparticles have been extensively studied to increase tumor uptake due to their good properties such as high loading efficacy, easy surface modification with targeting moieties, wide pharmacokinetic control, and easy control of size and shape. Among various nanoplatforms, liposomes have achieved clinical success due to their proven biocompatibility, biodegradability and low toxicity. However, there are currently no liposome preparations that can be used for diagnostic imaging in clinical practice, other than lipid-coated microbubbles used as ultrasound enhancers.

Enhancement of contrast agents has been largely hampered by the inevitable high uptake of nanoparticles into organs of the mononuclear phagocyte system (MPS), such as the liver and spleen. Tumor uptake may be improved with advanced nanotechnology. However, because uptake in the liver and spleen is often significantly higher than that in the tumor, only small improvements have been achieved in the tumor-to-background ratio, especially the tumor-to-liver and tumor-to-spleen ratios. Therefore, recent research has focused on achieving high contrast by reducing background noise instead of increasing tumor uptake. Specifically, the nanoparticle size was reduced to 5 nm or less, allowing the nanoparticles to be efficiently filtered by the renal system and rapidly excreted from the urinary tract, while minimizing uptake into MPS organs. Pre-targeting imaging strategies and clearance agents have been successfully applied to reduce background noise, especially in blood. However, they did not achieve the blood circulation time required for high tumor uptake by accelerating renal clearance. In addition, both strategies required challenging chemicals and a second injection step, limiting their application in routine clinical use. Therefore, selectively reducing background noise without affecting tumor uptake has not yet yielded satisfactory results.

Meanwhile, pancreatic cancer still remains a life-threatening cancer. While the five-year survival rate for pancreatic cancer, at about 10 percent, has barely increased over the past 40 years, survival rates for other cancers have doubled due to early detection and recently developed treatment tools. Pancreatic cancer is difficult to diagnose early. This is because the pancreas is located deep in the abdomen and is surrounded by other large organs. Pancreatic cancer less than 1 cm in size is rarely detected using conventional ultrasound, CT or MRI imaging. Since pancreatic cancer has low specificity, FDG-PET nuclear imaging is not efficient in diagnosing pancreatic cancer.

### SUMMARY OF INVENTION

### TECHNICAL PROBLEM

In the present invention, a novel radioactive compound containing an ester group in its structure was developed to provide an imaging strategy that dramatically improves the tumor-to-background ratio by utilizing the activity of esterase differentially expressed in tumors and MPS organs. Under the discovery that such novel radioactive compound shows particularly excellent visualization ability in the pancreas, which exhibits a low level of esterase activity as compared to the reticuloendothelial system such as the liver and spleen, the present invention has been completed.

Accordingly, the object of the present invention is to provide a novel radioactive compound selected from the group consisting of Formulas 1 to 8 or a pharmaceutically acceptable salt thereof: 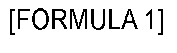 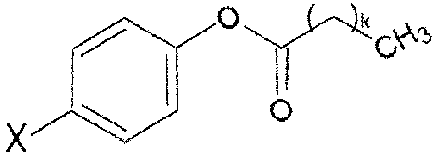 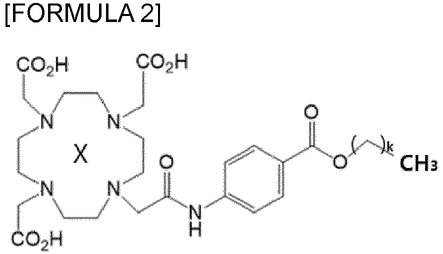 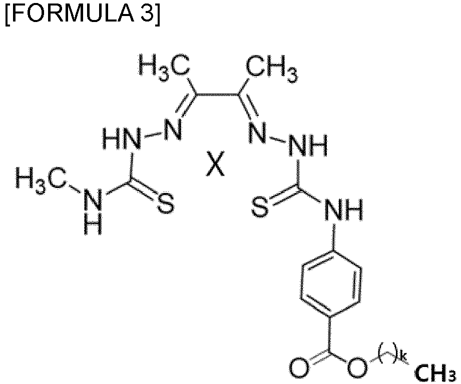 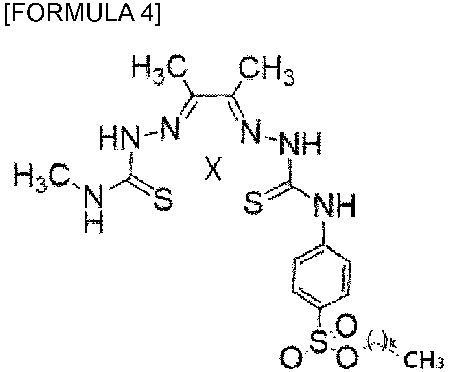 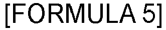 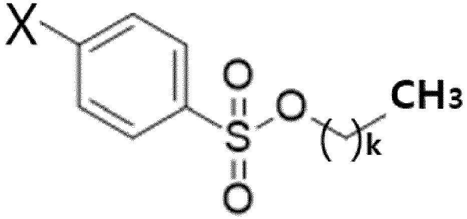 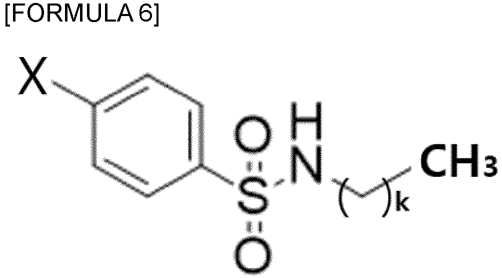 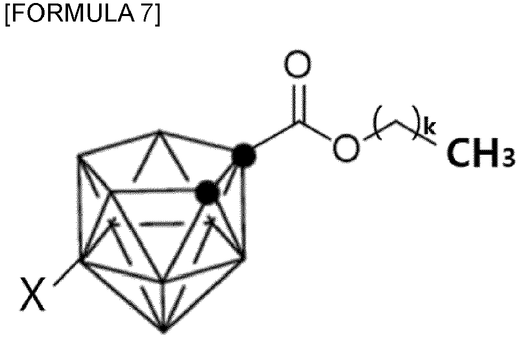 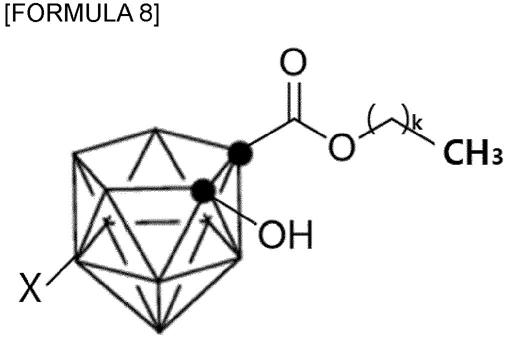 wherein, in the Formulas 1 to 8, X is a diagnostically active radioisotope selected from the group consisting of ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁵²Tb, ¹⁵⁵Tb, ²⁰¹ Tl, ²⁰³Pb, ¹⁸F, ⁷⁶Br, ⁷⁷Br, ¹²³I, ¹²⁴I and ¹²⁵I; or a therapeutically active radioisotope selected from the group consisting of ⁴⁷Sc, ⁶⁷Cu, ⁸⁹Sr, ⁹⁰Y, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹³¹I and ²¹¹At,
k is an integer from 5 to 30.

Another object of the present invention is to provide a liposome comprising the compound or a pharmaceutically acceptable salt thereof; and lipids.

Another object of the present invention is to provide a composition for *in vivo* imaging comprising the compound or a pharmaceutically acceptable salt thereof; or the liposome.

In addition, another object of the present invention is to provide a composition for *in vivo* imaging consisting of the compound or a pharmaceutically acceptable salt thereof; or the liposome.

In addition, another object of the present invention is to provide a composition for *in vivo* imaging consisting essentially of the compound or a pharmaceutically acceptable salt thereof; or the liposome.

Another object of the present invention is to provide a composition for diagnosing or treating a cancer comprising the compound or a pharmaceutically acceptable salt thereof; or the liposome.

In addition, another object of the present invention is to provide a composition for diagnosing or treating a cancer consisting of the compound or a pharmaceutically acceptable salt thereof; or the liposome.

In addition, another object of the present invention is to provide a composition for diagnosing or treating a cancer consisting essentially of the compound or a pharmaceutically acceptable salt thereof; or the liposome.

Another object of the present invention is to provide a compound of formula 9 or 10 or a pharmaceutically acceptable salt: 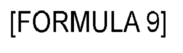 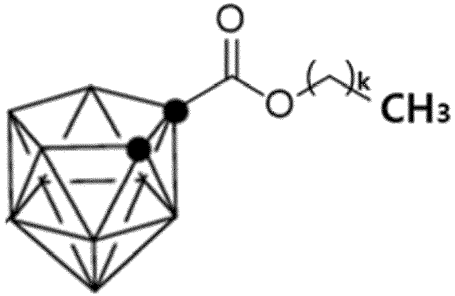 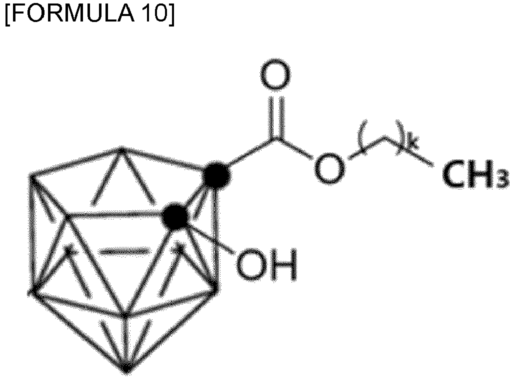
wherein, in the Formula 9 or 10,
k is an integer from 5 to 30.

Another object of the present invention is to provide a use of the compound of claim 1 or a pharmaceutically acceptable salt thereof, or the liposome of claim 2 for preparing a composition for in vivo imaging.

Another object of the present invention is to provide a method for in vivo imaging comprising the steps of:
i) administering the compound of claim 1 or a pharmaceutically acceptable salt thereof, or the liposome of claim 2 to a subject;
ii) waiting for a sufficient time to allow the compound or liposome to be introduced into the subject's body; and
iii) imaging the area where the compound or liposome is detected in the subject.

Another object of the present invention is to provide a use of the compound of claim 1 or a pharmaceutically acceptable salt thereof, or the liposome of claim 2 for preparing a composition for diagnosing a cancer.

Another object of the present invention is to provide a method for diagnosing a cancer comprising the steps of:
a) administering the compound of claim 1 or a pharmaceutically acceptable salt thereof, or the liposome of claim 2 to a subject;
b) waiting for a sufficient time to allow the compound or liposome to be introduced into cancer cells;
c) detecting proliferative cells by imaging the area where the compound or liposome is detected in the subject; and
d) diagnosing cancer when the compound or liposome is detected in the subject.

Another object of the present invention is to provide a use of the compound or a pharmaceutically acceptable salt thereof, or the liposome for preparing a composition for preventing or treating a cancer.

Another object of the present invention is to provide a method for treating neurodegenerative diseases, the method comprising administering an effective amount of a composition comprising the compound or a pharmaceutically acceptable salt thereof, or the liposome to a subject in need thereof.

### SOLUTION TO PROBLEM

In order to achieve the above-described objects of the present invention, the present invention provides a novel radioactive compound selected from the group consisting of Formulas 1 to 8 or a pharmaceutically acceptable salt thereof: 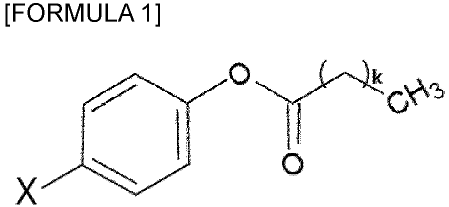 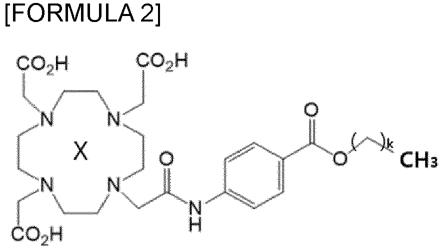 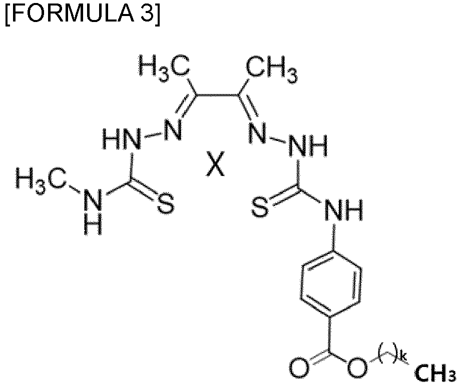 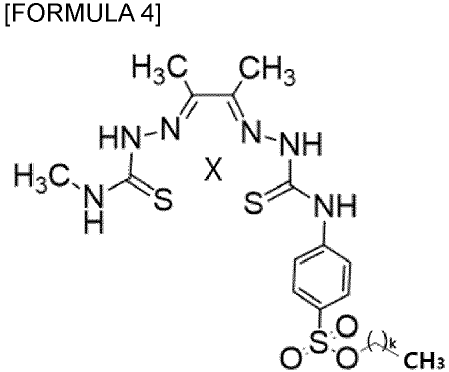 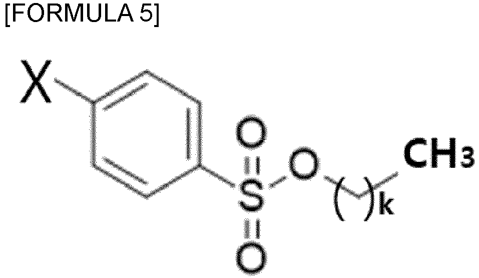 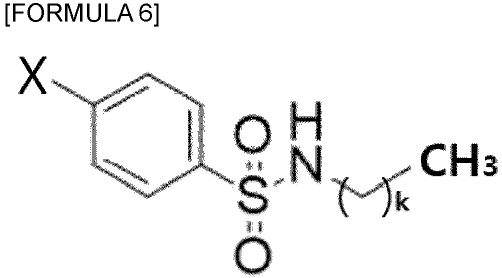 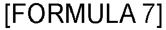 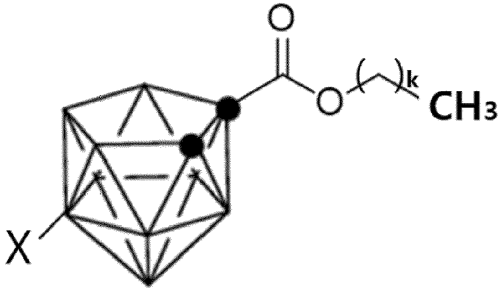 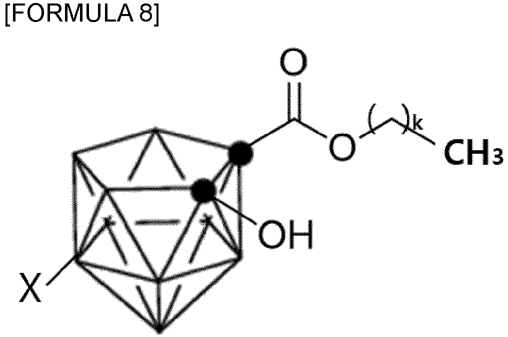
wherein, in the Formulas 1 to 8, X is a diagnostically active radioisotope selected from the group consisting of ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁵²Tb, ¹⁵⁵Tb, ²⁰¹ Tl, ²⁰³Pb, ¹⁸F, ⁷⁶Br, ⁷⁷Br, ¹²³I, ¹²⁴I and ¹²⁵I; or a therapeutically active radioisotope selected from the group consisting of ⁴⁷Sc, ⁶⁷Cu, ⁸⁹Sr, ⁹⁰Y, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹³¹I and ²¹¹At,
k is an integer from 5 to 30.

According to one embodiment of the present invention, it was confirmed that the compounds of Formulas 1 to 8 containing an ester group in their structures remained for a long time in pancreatic cancer tissue and the like, where esterase activity was relatively low, and were safe, and could effectively visualize tumors.

The compound may be used in the form of a pharmaceutically acceptable salt. As the salt, acid addition salts formed with various pharmaceutically and physiologically acceptable organic acids or inorganic acids are useful. Suitable organic acids may comprise, for example, carboxylic acid, phosphonic acid, sulfonic acid, acetic acid, propionic acid, octanoic acid, decanoic acid, glycolic acid, lactic acid, fumaric acid, succinic acid, adipic acid, malic acid, tartaric acid, citric acid, glutamic acid, aspartic acid, maleic acid, benzoic acid, salicylic acid, phthalic acid, phenylacetic acid, benzenesulfonic acid, 2-naphthalenesulfonic acid, methyl sulfuric acid, ethyl sulfuric acid and dodecyl sulfuric acid. Suitable inorganic acids may comprise, for example, hydrochloric acid, sulfuric acid and phosphoric acid.

When the radioisotopes bound or coordinated to Formulas 1 to 8 are diagnostically active radioisotopes, the compounds may be used as a tracer for diagnosing diseases in organs with relatively low esterase activity, such as pancreas. When the radioactive isotopes are therapeutically active isotopes, the compounds may be used as a therapeutic agent for treating diseases in organs with relatively low esterase activity, such as pancreas.

The present invention provides a liposome comprising the compound of claim 1 or a pharmaceutically acceptable salt thereof; and lipids.

The present invention may be characterized in that the lipids comprise (a) cholesterol; (b) 1,2-dipalmitoyl-sn-glycero-3-phosphocholine (DPPC); and (c) 1,2-distearoyl-*sn*-glycero-3-phosphoethanolamine-*N*-[methoxy(polyethylene glycol)-2000(DSPE-PEG₂₀₀₀)

The present invention may be distinguished from other liposome systems in that the compounds of Formulas 1 to 8 are loaded on the phospholipid bilayer of liposomes and are not directly conjugated to phospholipids. (That is, the contrast agent of the present invention is contained in liposomes in an unconjugated form with phospholipids.)

In the liposome of the present invention, the liposome acts as a carrier, and the movement of the contrast agents of Formulas 1 to 8 loaded on the liposome substantially plays a more important role in tumor diagnosis. In a tumor tissue, the liposome of the present invention itself or the contrast agent of Formulas 1 to 8 released from the liposome accumulates inside the tumor tissue, whereas, the reticuloendothelial system works in the way that the contrast substances of Formulas 1 to 8 are released from the absorbed liposome and are quickly discharged out of the body.

In order to impart this mode of action, the liposome is required to maintain adequate durability to withstand the body environment during the time the compound is transported (particularly, during it is transported to blood), and to be sensitive so that the compound may be released from the liposome at the target location or the liposome itself may be degraded. Accordingly, a unique lipid composition that may specifically impart the above-mentioned mode of action to the compounds of Formulas 1 to 8 used in the present invention is required.

Accordingly, the present invention may be characterized by the liposome composition capable of imparting such a mode of action in the body to the compounds of Formulas 1 to 8, wherein the liposome consists of (a) cholesterol; (b) DPPC; and (c) DSPE-PEG₂₀₀₀.

The lipids are characterized in that they have a molar ratio of (a):(b):(c) of 1:5-25:3-15, and preferably 1:8-20:4-13, more preferably 1:10-20:6-10, and most preferably 1:14-18:6-8.

More specifically, the lipids may have a molar ratio of (a):(b):(c) of 1:5, 6, 7, 8, 9, 10. 11, 12, 13, 14, 15, 16, 17, 18, 19, 20, 21, 22, 23, 24, or 25: 3, 4, 5, 6, 7, 8, 9, 10, 11, 12, 13, 14or15.

The liposomal contrast agent of the present invention having the lipid composition of (a), (b), and (c) is characterized by decreased uptake of the compounds represented by Formulas 1 to 8 in the reticuloendothelial system, and increased uptake and accumulation in tumors. In other words, the tumor-to-organ uptake ratio and accumulation rate (retention rate) of contrast agents in tumors were significantly increased, as compared to RES organs. That is, the production of a liposome system loaded with contrast agents of Formulas 1 to 8 with the unique lipid composition provided by the present invention enables efficient delivery, release, and accumulation of the contrast agents in a tumor-specific manner, while significantly reducing background noise in other tissues. In addition, when imaging using PET, the liposome of the present invention can significantly shorten the time for obtaining tumor images using compounds of Formulas 1 to 8.

Meanwhile, among the lipids constituting the liposome of the present invention, (c) DSPE-PEG₂₀₀₀ may be a portion of the total DSPE-PEG₂₀₀₀ composed in the liposome attached to folic acid.

That is, the liposome of the present invention may have a lipid composition consisting of: (a) cholesterol; (b) 1,2-dipalmitoyl-sn-glycero-3-phosphocholine (DPPC); (c-1) 1,2-distearoyl-*sn*-glycero-3-phosphoethanolamine-*N*-[methoxy(polyethylene glycol)-₂₀₀₀](DSPE-PEG₂₀₀₀); and (c-2) 1,2-Distearoyl-sn-glycero-3-phosphoethanolamine-*N*-[methoxy(polyethylene glycol)-2000]-folic acid (DSPE-PEG₂₀₀₀-folate).

At this time, the lipids are characterized in that they have a molar ratio of (a):(b):(c-1):(c-2) of 1 : 5-25 : 2-8 : 1-7, preferably 1 : 8-20 : 3-8 : 1-5, more preferably 1: 10-20 : 4-6 : 1-4, most preferably 1 : 14-18 : 4-6 : 1-3.

Specifically, the lipids may have a molar ratio of (a):(b):(c-1):(c-2) of 1 : 5, 6, 7, 8, 9, 10, 11, 12, 13, 14, 15, 16, 17, 18, 19, 20, 21, 22, 23, 24, or 25 : 2, 3, 4, 5, 6, 7, or 8 : 1, 2, 3, 4, 5, 6, or 7.

The liposome of the present invention having the lipid composition of (a), (b), (c-1) and (c-2) is characterized by decreased uptake (accumulation) in the reticuloendothelial system of the compounds represented by Formulas 1 to 8, and increased uptake (accumulation) in tumors overexpressing folate receptors.

The tumor overexpressing the folate receptor may be selected from the group consisting of pancreatic cancer, breast cancer, ovarian cancer, lung cancer, cervical cancer, colon cancer, melanoma, kidney cancer, brain tumor, myeloid leukemia, and head and neck cancer, but the present invention is not limited thereto.

Preferably, tumor or cancer in the present invention may indicate pancreatic cancer, breast cancer, colon cancer, ovarian cancer, cervical cancer, or melanoma. The lipid system of the present invention has a particularly high diagnostic value for pancreatic cancer, breast cancer, ovarian cancer, colon cancer, cervical cancer, and melanoma, more than other cancers, due to the unique histological characteristics thereof.

The use of the liposome of the present invention is not particularly limited as long as it is a means of imaging or imaging the compounds of Formulas 1 to 8, but the liposome, for example, may be used for optical imaging, positron emission tomography (PET), or single photon emission computed tomography (SPECT).

The present invention also provides a composition for in vivo imaging comprising the compound or a pharmaceutically acceptable salt thereof; or the liposome.

The compound or liposome according to the present invention may be administered systemically or locally to tumors, organs or tissues to be imaged. Typically, the compound or liposome is administered in a dose effective to achieve the desired optical image of the tumors, tissues or organs. These doses may vary widely depending on the tumors, tissues or organs receiving the imaging method, the imaging device to be used, and the like.

In some embodiments of the invention, the compounds or liposomes are used as, but not limited to, agents for in vivo optical imaging of tissues and organs in a variety of biomedical applications comprising imaging of tumors, tomographic imaging of organs, monitoring of the function of organs, coronary angiography, fluorescein endoscopy, laser-guided surgery, photoacoustic and phono fluorescence methods.

Examples of imaging methods comprise magnetic resonance imaging (MRI), MR spectroscopy, radiography, CT, ultrasound, planar gamma camera imaging, single photon emission computed tomography (SPECT), positron emission tomography (PET), and other nuclear medicine. Based imaging, optical imaging using visible light, optical imaging using luciferase, optical imaging using fluorophores, other optical imaging, imaging using near-infrared light, or imaging using infrared light.

Certain embodiments of the method of the present invention further comprise the step of imaging tissues during a surgical procedure in a subject.

Various techniques for imaging are known to those skilled in the art. Any of these techniques may be applied to the imaging method of the present invention to measure signals from a detectable label. For example, optical imaging is one imaging modality that is widely accepted in certain areas of medicine. Examples comprise optical labeling of cellular components and angiography such as fluorescein angiography and indocyanine green angiography.

Imaging modalities that provide information on the cellular level, such as cell viability, comprise positron emission tomography (PET) and single-photon emission computed tomography (SPECT). In PET, the patient absorbs or is injected with a radioactive material that emits protons that may be monitored as the substance moves through the body.

Single photon emission computed tomography, or SPECT, is closely related to PET. The main difference between the two is that SPECT uses radioactive tracers that emit high-energy photons instead of substances that emit protons.

For PET, compounds labeled with proton emitters such as ¹¹C, ¹³N, ¹⁵O, ¹⁸F, ⁸²Rb, ⁶²Cu and ⁶⁸Ga may typically be used. For SPECT, the compounds may be labeled with proton emitters such as ^{99m}Tc, ²⁰¹Tl, ⁶⁷Ga and ¹¹¹In.

Noninvasive fluorescence imaging in animals and humans also provides in *vivo* diagnostic information and can be used in a wide variety of clinical specialties. For example, techniques involving simple observations have been developed over the years, ranging from UV excitation of fluorophores to complex spectroscopic imaging using advanced instruments. For example, specific devices or methods known in the art for *in vivo* detection of fluorophores or fluorescent proteins include, but are not limited to, *in vivo* near-line fluorescence, *in vivo* fluorescence imaging systems, flying spot scanners, and the like.

Other methods or devices for detecting optical responses comprise, but are not limited to, visual observation, and signal amplification using CCD camera, video camera, photographic film, laser scanning device, fluorometer, photosensitive semiconductor device, quantum instrument, extra fluorescence microscope, scanning microscope, flow cytometer, fluorescence microplatelet reader, or photomultiplier tube.

The compound according to one aspect is novel, and the compound, the liposome, or the composition comprising them according to another aspect can create a clear image *in vivo* using radioactive isotopes, thereby producing the effect of diagnosing a disease. Furthermore, when performing a pre-targeted imaging technique using the compound, an image clearly showing only the tumor without any background signal can be obtained.

The present invention provides a composition for diagnosing cancer and a composition for treating cancer, comprising the compound or a pharmaceutically acceptable salt thereof; or the liposome.

It is also within the scope of the present invention that the compounds of the present invention be used in theranostic methods. The concept of diagnostics is to combine therapeutic agents with corresponding diagnostic tests that can increase the clinical use of therapeutic drugs. The concept of diagnostics is increasingly attractive, and it is widely regarded as its core for doctors to identify patients who may benefit from a given therapy and help patients avoid unnecessary treatment, thereby improving the efficiency of drug treatment.

The concept of diagnostics is to combine diagnostic tests and therapeutics that enable doctors to identify patients who will benefit most from a given therapy. In one embodiment and as preferably used herein, the compounds of the present invention are used for the diagnosis of patients, i.e., for the identification and localization of, in addition to the primary tumor mass, the potential local and distant metastases thereof. In addition, tumor volume may be measured particularly using three-dimensional diagnostic modalities such as SPECT and PET. Only patients who can benefit from a given therapy are selected for a particular therapy, thereby avoiding unnecessary treatment. In one particular embodiment, chemically identical tumor-targeted diagnostic agents, preferably imaging diagnostic agents for scintigraphy, PET or SPECT, and radiotherapy, are applied. These compounds differ only in the radionuclide and therefore usually have very similar, if not identical, pharmacokinetic profiles. This may be realized using chelating agents and diagnostic or therapeutic radiometals. Alternatively, this may be realized using radiolabeling with diagnostic or therapeutic radionuclides and precursors for radiolabeling. In one embodiment, diagnostic imaging is preferably used for quantification of radiation of diagnostic radionuclides and follow-up dosimetry known to those skilled in the art and prediction of drug concentration in the tumor, as compared to organs of vulnerable side effects. A truly individualized drug dosing regimen for the patient is therefore achieved.

In one embodiment and as preferably used herein, the theranostic method may be realized with only one therapeutically active compound such as a compound of the invention labeled with a radionuclide that emits, in addition to diagnostically detectable radiation (e.g., positrons and gamma rays), therapeutically effective radiation (e.g., electrons and alpha particles).

The pharmaceutical composition of the present invention, together with a pharmaceutically acceptable carrier, may be formulated in various ways, in addition to the fusion protein, depending on the route of administration by methods commonly known in the art. "Pharmaceutically acceptable" indicates a non-toxic substance that is physiologically acceptable, and, when administered to humans, neither inhibits the action of the active ingredient nor usually causes gastrointestinal upset, allergic reactions such as dizziness, or similar reactions. The carrier comprises all types of solvents, dispersion media, oil-in-water or water-in-oil emulsions, aqueous compositions, liposomes, microbeads and microsomes.

The route of administration may be oral or parenteral. Parenteral administration methods may comprise, but are not limited to, intranasal, intravenous, intramuscular, intraarterial, intramedullary, intrathecal, intracardiac, transdermal, subcutaneous, intraperitoneal, intranasal, enteral, topical, sublingual or intrarectal administration.

When the pharmaceutical composition of the present invention is administered orally, the pharmaceutical composition of the present invention, together with a suitable carrier for oral administration, may be formulated according to methods commonly known in the art in the forms of powder, granule, tablet, pills, sugar-coated tablets, capsules, liquids, gels, syrups, suspensions, wafers, and the like. Examples of suitable carriers may comprise saccharides such as lactose, dextrose, sucrose, sorbitol, mannitol, xylitol, erythritol and maltitol; starches such as corn starch, wheat starch, rice starch and potato starch; celluloses such as cellulose, methyl cellulose, sodium carboxymethylcellulose and hydroxypropyl methyl cellulose; and fillers such as gelatin and polyvinylpyrrolidone. In addition, in some cases, cross-linked polyvinylpyrrolidone, agar, alginic acid, or sodium alginate may be added as a disintegrant. Furthermore, the pharmaceutical composition may further comprise anticoagulants, lubricants, wetting agents, fragrances, emulsifiers, and preservatives.

In addition, when administered parenterally, the pharmaceutical composition of the present invention, together with a suitable parenteral carrier, can be formulated according to a method known in the art in the form of injections, transdermal administration and nasal inhalation. In the case of the injection, it must be sterilized and protected from contamination of microorganisms such as bacteria and fungi. For injection, examples of suitable carriers may include, but are not limited to, water, ethanol, polyols (e.g., glycerol, propylene glycol and liquid polyethylene glycol, etc.), mixtures thereof, and/or a solvent or dispersion medium containing vegetable oil. More preferably, suitable carriers, such as, Hanks' solution, Ringer's solution, phosphate buffered saline (PBS) or sterile water for injection with triethanolamine, isotonic solutions such as 10% ethanol, 40% propylene glycol and 5% dextrose, etc. can be used. In order to protect the injection from microbial contamination, it may further include various antibacterial and antifungal agents such as parabens, chlorobutanol, phenol, sorbic acid, thimerosal, and the like. In addition, in most cases, the injection may further contain an isotonic agent such as sugar or sodium chloride.

Transdermal administration agents comprise forms such as ointments, creams, lotions, gels, external solutions, paste preparations, linear preparations, and aerosol preparations. In the above, "transdermal administration" means topically administering a pharmaceutical composition to the skin so that an effective amount of the active ingredient contained in the pharmaceutical composition is delivered into the skin. For example, the pharmaceutical composition of the present invention may be prepared in an injectable formulation and administered by lightly pricking the skin with a 30-gauge thin injection needle or applying it directly to the skin. These formulations are described in Formulas generally known in pharmaceutical chemistry.

For inhalation administration, the compounds used according to the present invention may conveniently be delivered in the form of an aerosol spray from a pressurized pack or nebulizer, by using a suitable propellant (e.g., dichlorofluoromethane, trichlorofluoromethane, dichlorotetrafluoroethane, carbon dioxide and other suitable gas). In the case of pressurized aerosols, the dosage unit may be determined by providing a valve that delivers a metered amount. For example, gelatin capsules and cartridges for use in inhalers or insufflators may be formulated to contain a compound and a powder mixture of a suitable powder base such as lactose and starch.

For other pharmaceutically acceptable carriers, those known in the art may be referred to.

In addition, the pharmaceutical composition according to the present invention may further comprise one or more buffers (e.g., saline and PBS), carbohydrates (e.g., glucose, mannose, sucrose and dextran), antioxidants, bacteriostatic agents, chelating agents (e.g., EDTA and glutathione), adjuvants (e.g., aluminum hydroxide), suspending agents, thickening agents and/or preservatives.

In addition, the pharmaceutical compositions of the present invention may be formulated using methods known in the art to provide rapid, sustained or delayed release of the active ingredient after administration to a mammal.

In addition, the pharmaceutical composition of the present invention may be administered in combination with known substances that are effective in preventing or treating cancer.

The present invention provides the use of the compound or a pharmaceutically acceptable salt thereof; or the liposome, for preparing a composition for *in vivo* imaging.

The present invention provides a method for *in vivo* imaging comprising the steps of:
i) *in vivo* administering the compound or a pharmaceutically acceptable salt thereof, or the liposome to a subject;
ii) waiting for a sufficient time to allow the compound or liposome to be introduced into the subject's body; and
iii) imaging the area where the compound or liposome is detected in the subject.

The present invention provides the use of the compound or a pharmaceutically acceptable salt thereof; or the liposome, for preparing a composition for diagnosing a cancer.

The present invention provides a method for diagnosing a cancer comprising the steps of:
a) administering the compound or a pharmaceutically acceptable salt thereof, or the liposome to a subject;
b) waiting for a sufficient time to allow the compound or liposome to be introduced into cancer cells;
c) detecting proliferative cells by imaging the area where the compound or liposome is detected in the subject; and
d) diagnosing cancer when the compound or liposome is detected in the subject.

The present invention provides the use of the compound or a pharmaceutically acceptable salt thereof; or the liposome, for preparing a composition for treating a cancer.

The present invention provides a method for treating a cancer by administering an effective amount of a composition comprising the compound or a pharmaceutically acceptable salt thereof, or the liposome to a subject in need thereof.

The term "effective amount" of the present invention refers to an amount that exhibits an effect of improving, treating, detecting, diagnosing a cancer, or inhibiting or reducing the progression of a cancer, when administered to a subject. In addition, the term "individual" may be an animal, preferably a mammal, particularly an animal comprising a human being, and may also be a cell, tissue, or organ derived from an animal. The subject may be a patient in need of the effect.

The term "treatment" of the present invention comprehensively refers to improving a cancer or a symptom caused by a cancer, may comprise curing, substantially preventing, or improving the condition of the disease, and comprises mitigating, curing, or preventing one or most of the symptoms caused by the disease, but the present invention is not limited thereto.

As used herein, the term "comprising" is used in the same sense as "including" or "characterized by." The composition or method according to the present invention does not exclude additional components or method steps not specifically mentioned. In addition, the term "consisting of" refers to excluding additional elements, steps or components not separately described. The term "essentially consisting of" means that, in addition to the described materials or steps, materials or steps that do not substantially affect the basic characteristics thereof may be contained in the scope of a composition or method.

### ADVANTAGEOUS EFFECTS OF INVENTION

A novel compound of chemical Formulas 1 to 8 according to the present invention exhibits selectivity according to the difference in activity of esterase for each organ due to ester residue in a structure, and thus is extremely effective in visualizing pancreatic cancer, etc.

### BRIEF DESCRIPTION OF DRAWINGS

FIGS. 1A to 1E show the structure of the radioactive compound prepared in Example 1 of the present invention and the liposome comprising (FIG. 1A), the synthesis method of the radioactive compound (FIG. 1B), the radio-TLC profile of the radioactive compound (FIG. 1C), the results of measuring the diameter of [¹³¹I]HIB-liposome ([¹³¹I]**L1**) (FIG. 1D) and the Cryo-TEM image of HIB-liposome (L1) (FIG. 1E).
FIGS. 2A to 2D show the results of confirming tumor uptake and *in vivo* excretion of the radioactive compounds prepared in Example 1 of the present invention.
FIGS. 3A to 3F are diagrams showing the results of metabolic studies of radioactive compounds prepared in Example 1 of the present invention.
FIGS. 4A to 4I are diagrams showing the results of observing *in vivo* dynamics after administering folic acid-conjugated [^{124/131}I]HIB-liposome (L4) to a xenograft animal model transplanted with pancreatic cancer cells.
FIG. 5 shows the results of confirming biodistribution 7 hours after injecting ⁶⁴Cu-DOTA-Benzene-HIB into mice to determine the *in vivo* excretion rate of ⁶⁴Cu-DOTA-Benzene-HIB.
FIG. 6 shows the results of PET/CT imaging performed for 3.5 and 7 hours after injection of ⁶⁴Cu-DOTA-Benzene-HIB.
FIG. 7 shows the results of confirming tumor uptake after injecting ⁶⁴Cu-DOTA-Benzene-HIB-Liposome into a xenograft mouse model transplanted with CT26 tumor cells.
FIG. 8 shows the results of PET/CT imaging performed 16 hours after injecting ⁶⁴Cu-DOTA-Benzene-HIB-Liposome into a xenograft mouse model transplanted with CT26 tumor cells.
FIG. 9 shows the results of confirming *in vivo* excretion rate 24 hours after injecting ⁶⁴Cu-DOTA-Benzene-HIB-Liposome into a mouse to determine the in vivo excretion rate of ¹⁷⁷Lu-DOTA-Benzene-HIB-Liposome.
FIG. 10 shows the results of confirming biodistribution 24 hours after injecting ¹⁷⁷Lu-DOTA-Benzene-HIB-Liposome into a tumor mouse model to confirm tumor uptake of ¹⁷⁷Lu-DOTA-Benzene-HIB-Liposome.
FIGS. 11 to 13 show the results of confirming biodistribution 24 hours after injecting ¹³¹I-HIB-sulfate-Liposome into a tumor mouse model to confirm tumor uptake of ¹³¹I-HIB-sulfate-Liposome.
FIG. 14 shows the results of confirming biodistribution 24 hours after injecting ¹³¹I-*nido*-Carborane-HIB into mice to determine the *in vivo* excretion rate of ¹³¹I-*nido-*Carborane-HIB.
FIG. 15 shows the results of confirming the *in vivo* excretion process of FIG. 15 shows the results of confirming the in vivo excretion process of ¹³¹I-*nido*-Carborane-HIB through PET/CT analysis.

### MODE FOR INVENTION

Hereinafter, the present invention will be described in detail by the following embodiments. However, the following embodiments are only for illustrating the present invention, and the present invention is not limited thereto.

### I. Example 1

### Methods

### 1. Synthesis of IPH (4-iodophenyl heptadecanoate)

A mixture of p-iodophenol (176 mg, 0.83 mmol) and heptadecanoic acid (270 mg, 0.91 mmol) in dichloromethane (20 mL) was added to 1-ethyl-3-(3-dimethylaminopropyl) carbodiimide (EDC) (174 mg, 0.91 mmol) and 4-dimethylaminopyridine (DMAP) (121 mg, 1 mmol), and the mixture was stirred at room temperature overnight (Scheme S1a). The formation of IPH was confirmed by TLC (silica TLC, Hexane/EtOAc 20:1). The reaction mixture was then washed with water, and the collected organic layer was dried over MgSO₄ and evaporated to dryness. The crude product was purified by column chromatography to give IPH as a white solid (276 mg, 73%).

¹H NMR (400 MHz, CDCl₃): δ 0.88 (t, *J* = 7.2 Hz, 3 H), 1.21-1.41 (m, 26 H), 1.70-1.77 (m, 2 H), 2.53 (t, *J* = 7.6 Hz, 2 H), 6.84 (d, *J* = 10 Hz, 2 H), 7.67 (d, *J* = 9.6 Hz, 2 H); ¹³C NMR (100 MHz, CDCl₃): δ 14.1, 22.7, 24.9, 29.1, 29.2, 29.4, 29.5, 29.59, 29.64, 29.67, 29.68, 29.70, 31.9, 34.3, 89.7, 123.8, ¹²⁴.1, 138.4, 150.6, 171.9; HRMS (FAB): m/z calcd. for C₂₃H₃₈IO₂ [M+H]⁺: 473.1916; found: 473.0576.

### 2. Synthesis of 4-(tributylstannyl)phenyl heptadecanoate

Hexabutylditin (0.38 g, 0.66 mmol) and tetrakis(triphenylphosphine)palladium (2.6 mg) were sequentially added to a stirred solution of IPH (0.21 g, 0.44 mmol) in anhydrous toluene (10 mL), and the mixture was refluxed for 16 h (Scheme S1a). The black-colored mixture was then allowed to cool to room temperature and filtered. Toluene was evaporated in vacuo and the resulting black oil was purified by column chromatography to afford 4-(tributylstannyl)phenyl heptadecanoate as a colorless oil (178 mg, 63% yield).

¹H NMR (500 MHz, CDCl₃): δ 0.89-1.00 (m, 20 H), 1.28-1.39 (m, 32 H), 1.48-1.67 (m, 6 H), 2.38 (m, 2 H), 6.64 (d, *J* = 8.5 Hz, 2 H), 7.53 (d, *J* = 8.5 Hz, 2 H); ¹³C NMR (100 MHz, CDCl₃): δ 13.7, 14.1, 22.7, 25.7, 27.5, 29.37, 29.42, 29.6, 29.70, 29.71, 30.6, 31.9, 32.7, 117.9, 122.3, 138.4, 155.8, 170.6; HRMS (FAB): m/z calcd. for C₃₅H₆₄O₂Sn [M+H]⁺: 637.4007; found: 637.4027.

### 3. Synthesis of 4-(tributylstannyl)phenyl heptadecanoate)

A mixture of *p*-iodophenol (161 mg, 0.74 mmol) and 1-iodohexadecane (312 mg, 0.89 mmol) in N,N-dimethylformamide (5 mL) was added to K₂CO₃ (126 mg, 1 mmol), and the mixture was stirred at room temperature overnight (Scheme S1b). The formation of HIB-ether was confirmed by TLC (silica TLC, Hexane/EtOAc 20:1). The mixture was then diluted with dichloromethane, filtered, evaporated in vacuo, and extracted with dichloromethane. The organic layer was collected, dried over MgSO4, and evaporated to dryness. The crude residue was purified by column chromatography to give HIB-ether as a white solid in 52% yield (169 mg).

¹H NMR (400 MHz, CDCl₃): δ 0.88 (t, *J* = 6.8 Hz, 3 H), 1.20-1.46 (m, 26 H), 1.72-1.78 (m, 2 H), 3.88 (t, *J* = 6.8 Hz, 2 H), 6.64 (d, *J* = 10 Hz, 2 H), 7.51 (d, *J* = *10* Hz, 2 H); ¹³C NMR (100 MHz, CDCl₃): δ 14.1, 22.5, 26.0, 29.2, 29.4, 29.58, 29.61, 29.68, 29.72, 31.9, 68.0, 82.4, 116.9, 138.1, 159.0; HRMS (FAB): m/z calcd. for C₂₂H₃₇IO [M+Na]+ 467.1787; found 467.1755.

### 4. Synthesis of tributyl(4-(hexadecyloxy)phenyl)stannane

Hexabutylditin (0.25 g, 0.44 mmol) and tetrakis(triphenylphosphine)palladium (1.2 mg) were sequentially added to a stirred solution of 1-(hexadecyloxy)-4-iodobenzene (0.13 g, 0.29 mmol) in anhydrous toluene (10 mL), and the mixture was refluxed for 16 h (Scheme S1b). The black-colored mixture was then allowed to cool to room temperature and filtered. Toluene was removed in vacuo and the resulting black oil was purified by column chromatography (silica TLC, Hexane/EtOAc 20:1) to afford tributyl(4-(hexadecyloxy)phenyl)stannane as a colorless oil (103 mg, 58% yield).

¹H NMR (500 MHz, CDCl₃): δ 0.90-1.00 (m, 21 H), 1.28-1.36 (m, 34 H), 1.48-1.07 (m, 3 H), 3.69 (d, *J* = 6.5 Hz, 2H), 6.64 (d, *J* = 8.5 Hz, 2 H), 7.52 (d, *J* = 8.5 Hz, 2 H); ¹³C NMR (125 MHz, CDCl₃): δ 13.7, 14.1, 22.7, 25.7, 29.0, 29.3, 29.37, 29.44, 29.6, 29.66, 29.67, 29.70, 30.6, 31.9, 32.8, 63.1, 115.4, 118.2, 139.7, 160.0; HRMS (FAB): m/z calcd. for C₃₄H₆₄OSn [M+Na]+: 631.3877; found: 631.3285.

### 5. Synthesis of [^{124/131}I]HIB (1), [^{124/131}I]IPH (2), or [^{124/131}I]HIB-ether (3)

A solution of 100 µg of hexadecyl-4-tributylstannylbenzoate, 4-(tributylstannyl)phenyl heptadecanoate, or tributyl(4-(hexadecyloxy)phenyl)stannane in acetic acid was mixed with 1 N HCl, [^{124/131}I]Nal (18.5-185 MBq), and 3% H₂O₂. After stirring at 70 °C for 30 min, the reaction was terminated with NaHSOs. The reaction solvent and free radioiodine were removed in vacuo at 60 °C. Afterward, radiolabeled [^{124/131}I]HIB (1), [^{124/131}I]IPH (2), or [^{124/131}I]HIB-ether (3) was extracted by acetonitrile and evaporated to dryness in vacuo. The purified radiotracers (1-3) were then reconstituted in chloroform for the following liposome preparation, and their radiochemical purity were analyzed by radio-TLC using a radio-TLC imaging scanner (AR-2000, Bioscan, USA).

### 6. Preparation of liposomes (L1 to L4) loaded with radiotracer

In order to optimize the liposomal components and increase the selectivity of tumor uptake, liposomal compositions consisting of different DPPC, DPPG, cholesterol, and DSPE-mPEG₂₀₀₀ molar ratios were prepared in chloroform.

In brief, [^{124/131}I]HIB (1) was mixed with the liposomal components in a round bottom flask and then evaporated in vacuo for 20 min to form a lipid thin film. Afterward, the lipid film was hydrated upon treatment with phosphate-buffered saline (PBS; 1 mL) at the transition temperature of the lipid for 20 min. Then, ~1 mL of the lipid dispersion was extruded 21 times through polycarbonate membrane filters (Whatman, Kent, UK) with different pore sizes (1000, 400, 200, and 100 nm) by a mini extruder (Avanti Polar lipids, USA). The synthesized liposomes were purified and concentrated by centrifugal filtration (YM-100, Millipore, St. Louis, MO, USA). The molar ratio of DPPC (587 µg, 0.8 µmol), cholesterol (19 µg, 0.05 µmol), and DSPE-mPEG₂₀₀₀ (977 µg, 0.35 µmol) for the preparation of [^{124/131}I]HIB-liposome (L1), [^{124/131}I]IPH-liposome (L2) and [^{124/131}I]HIB-ether-liposome (L3) was 16:1:7.

Folate-conjugated [^{124/131}I]HIB-liposome for pancreatic cancer imaging was prepared by partially replacing DSPE-mPEG₂₀₀₀ with DSPE-mPEG₂₀₀₀-folate without changing the molar ratio of DPPC and cholesterol.

The optimal liposomal composition of L4 was 16:1:5:2 corresponding to 587 µg of DPPC (0.8 µmol), 19 µg of cholesterol (0.05 µmol), 698 µg of DSPE-mPEG₂₀₀₀ (0.25 µmol), and 323 µg of DSPE-mPEG₂₀₀₀-folate (0.1 µmol).

### 7. Culturing of cells and Animals

Murine colorectal carcinoma CT26 cells were cultured in RPMI 1640 medium supplemented with 10% FBS and 1% penicillin-streptomycin at 37 °C under 5% CO₂, as recommended by ATCC. Two human pancreatic cancer cells lines were used, PANC-1 (FR+) and MIA PaCa-2 (FR-) (FR: folate receptor). All animal experiments were conducted in line with the Animal Ethics Committee requirements of Kyungpook National University (approval nos. 2014-0154, 2017-0096, and 2020-0079). BALB/c mice (male, 5 weeks, 16-20 g) were purchased from Hyochang Science (Daegu, Korea) and BALB/c nude mice (male, 5 weeks, 16-19 g) were purchased from Orient Bio (Seongnam, Korea). All animals were maintained at 20-24 °C for 12-h day and night cycles under sufficient supply of water and commercial feed.

### 8. Tumor mouse models

CT26 cells (5 × 10⁶ cells/mouse) were subcutaneously inoculated into the right flank of BALB/c mice. When the tumor reached a diameter of ~6-8 mm (12 days after inoculation), the CT26 tumor models were used for *in vivo* studies. In order to evaluate the targeting ability of L4 based on the FR expression level in pancreatic cancer cells, PANC-1 (FR+) and MIA PaCa-2 (FR-) cells (5 × 10⁶ each) were inoculated into the right and left flanks of BALB/c nude mice, respectively. When the tumor of both sides reached a diameter of 4-6 mm, pancreatic xenograft models were subjected to positron emission tomography (PET) imaging.

PANC-1 and MIA PaCa-2 cells were inoculated separately only on the right mouse flank (5 × 10⁶ cells/mouse) to establish tumor-bearing xenograft models for biodistribution studies. The orthotopic pancreatic tumor model was prepared by the inoculation of PANC-1/Luc+ cells (1 × 10⁶ cells in 50 µL DMEM medium) into the pancreatic tail of BALB/c nude mice.59, 60 At 13 days post-inoculation, bioluminescent imaging with D-luciferin (60 mg/kg) was performed using IVIS Lumina-XR (PerkinElmer, Waltham, USA) to verify the establishment of the tumor.

### 9. Whole body clearance

To quantitatively monitor the clearance rate of L1, L2, and L3, CT26 tumor-bearing BALB/c mice were intravenously injected with each liposome (6.7-9.3 MBq/mouse). The whole-body radioactivity was measured using a dose calibrator (CRC-25R, Capintec, Ramsey, NJ, USA) at 10 min, 20 min, 30 min, 45 min, 1 h, 1.5 h, 2 h, 3 h, 4 h, 6 h, 8 h, 12 h, and 24 h.

### 10. Metabolism studies

The in vitro metabolism studies were performed using 25 U/mL solutions of porcine liver esterase in PBS. [¹³¹I]HIB (1), [¹³¹I]IPH (2), or [¹³¹I]HIB-ether (3) (3.7 MBq each) was also dissolved in PBS with 10% DMSO. Esterase (2.5 U) was then added to each radiotracer solution, and the solution was gently shaken at 37 °C. The hydrolysis rate of each radiotracer was measured by radio-TLC (silica TLC, Hexane/EtOAc 20:1) at 1 min, 5 min, 10 min, 1 h, 2 h, 4 h, 16 h, and 24 h.

For the in vitro metabolic studies with mouse liver S9 fraction and microsome, 1-3 (3.7 MBq) were dissolved in PBS with 10% DMSO and mixed with the mouse liver S9 fraction (0.2 mg in 10 µL) or the microsome (0.2 mg in 10 µL) with NADPH (0.25 mM). The reaction was performed upon shaking at 550 rpm at 37 °C. The hydrolysis rate of each radiotracer was measured by radio-TLC for up to 24 h.

*Ex vivo* studies to determine the cleavage rates of the examined radiotracers in different organs were performed using the homogenates of mouse tissues collected from CT26 tumor models. Liver, spleen, and tumor tissues were collected and weighted. After the addition of ice-cold PBS solution at a ratio of 1:1 (w/w), the tissues were homogenized with a homogenizer (Ultra-Turrax, IKA, Selangor, Malaysia) under ice-cold conditions. The crude homogenate was then centrifuged at 2,000 g, and the sediment was discarded. Each supernatant (300 µL), referred to as tissue homogenate, was immediately mixed with each radiotracer (3.7 MBq) dissolved in PBS with 10% DMSO. The reaction was then performed upon shaking at 550 rpm at 37 °C, and the hydrolysis rate of each radiotracer was measured by radio-TLC for up to 24 h. To analyze the urinary and fecal excretion of the radioactive metabolites, [¹³¹I]HIB-liposome ([¹³¹I]L1) (17-21 MBq in 200 µL PBS with 10% DMSO) was intravenously injected into normal BALB/c mice (n = 6), which were kept in metabolic cages (two mice/cage). Urine and fecal samples were collected separately at 0.5, 1, 2, 4, 8, 16, and 24 h post-injection, and their radioactivity was measured with a dose calibrator (CRC-Ultra; Capintec, Florham Park, NJ, USA). A urine sample collected at 3 h post-injection of [¹³¹I]L1, which showed the highest radioactivity among the collected samples, was used to analyze the metabolite of [¹³¹I]HIB by radio-TLC (silica TLC, dichloromethane/acetic acid 9:1) and compare its radio-TLC peaks with free I-¹³¹ and 4-[¹³¹I]iodobenzoic acid. [¹³¹I]HIB (3.7 MBq in 200 µL acetonitrile) was treated with 1 M NaOH (200 µL) at 90 °C for 3 h to hydrolyze [¹³¹I]HIB into 4-[¹³¹I]iodobenzoic acid (3.3 MBq). The same urine sample was further analyzed by HPLC [Luna C8 column (4.6 × 50 mm, 5 µm), 0.1% TFA/H₂O (solvent A), and 0.1% TFA/acetonitrile (solvent B)] using a gradient of 10-100% B in 5 min and 10% B for 27 min, which continued for up to 45 min at a flow rate of 1 mL/min.

### 11. Biodistribution studies

The biodistribution of [¹³¹I]HIB-liposome (L1), [¹³¹I]IPH-liposome (L2) and [¹³¹I]HIB-ether-liposome (L3) was compared using CT26 tumor-bearing BALB/c mice. The three liposomes were intravenously injected into CT26 tumor models via the tail vein (0.5-0.8 MBq/mouse), and the tumor-bearing mice were anesthetized with 1-2% isoflurane in O2 and euthanized at 1, 4, and 24 h post-injection. The organ tissues were then collected, weighed immediately, and measured in a γ-counter (Wallach, Turku, Finland). The biodistribution data were expressed as a percentage of the injected dose per gram (%ID/g).

To determine the biodistribution of folate-[¹³¹I]HIB-liposome (L4), 0.5-1.0 MBq/mouse was intravenously injected into the tail vein of pancreatic xenograft models bearing PANC-1 (FR+) or MIA PaCa-2 (FR-) tumors. At 24 h post-injection, the mice were euthanized under anesthesia, and their organs and tissues were collected, weighed immediately, and measured in a γ-counter. The biodistribution of L4 (0.5-1.0 MBq) in an orthotopic pancreatic tumor model at 1, 4, and 24 h post-injection was evaluated following the same process.

### 12. Animal Imaging

The PET images of [¹²⁴I]L1-L3 in CT26 tumor models were obtained by an Inveon PET/ computed tomography (CT) scanner (Siemens, Knoxville, USA) using the Inveon Acquisition Workplace software (version 1.5). The imaging parameters were as follows: detector pixel spacing, 1.6 × 1.6 mm2; field of view, 10 × 12.7 cm (trans axial × axial); γ-ray energy window, 350-650 keV. The PET images were reconstructed using the ordered-subsets expectation maximization 3D maximum a posteriori (OSEM3D/MAP) algorithm and then converted to the format of digital imaging and communications in medicine using the Inveon Research Workplace 4.0.

Finally, the PET images were analyzed using the Mediso Interview Fusion software package. The radioactivity in PET images was calibrated in standardized uptake values. All other PET/CT images were obtained by a nanoScan PET/CT scanner (PET 82S, Mediso, Budapest, Hungary) with the following imaging parameters: detector pixel spacing, 0.9 × 0.9 mm2; field of view, 8 × 10 cm (trans axial × axial); 1-5 coincidence mode with 5 ns coincidence window; γ-ray energy window, 400-600 keV. PET scanning was performed for 20 min at 1 h and 4 h post-injection, but the scanning time was increased to 45 min for images at 24 h post-injection.

During PET scanning, mice were anesthetized with 1-2% isoflurane in O₂ and set in the prone position. CT scanning was also conducted without a contrast agent for ~10 min to obtain an anatomical reference and to correct the attenuation of the PET images. The PET images were then reconstructed in 1-3 coincidence mode in four iterations with three subsets using the Mediso Tera-Tomo 3D iterative algorithm with corrections for the interaction depth, radionuclide decay, detector normalization, crystal dead time, and attenuation. The PET and CT images were automatically co-registered and analyzed using the Mediso Interview Fusion software package. The radioactivity in the PET images was calibrated in standardized uptake values. The radioactivity in PET images was calibrated in standardized uptake values.

To compare the tumor targeting and clearance pattern of [¹²⁴I]L1-L3, CT26 tumor models were intravenously injected via the tail vein with three different liposomes (6.7-9.3 MBq/mouse), and PET images were obtained at 1, 4, and 24 h post-injection. [¹²⁴I]HIB (7.4 MBq in 200 µL PBS with 10% DMSO) and 4-[¹²⁴I]iodobenzoic acid (7.4 MBq in 200 µL PBS) were also intravenously injected into CT26 tumor models and the corresponding PET images were recorded at 1, 4, and 24 h post-injection to verify their rapid clearance and inability to target the tumor.

For PET/CT imaging of the pancreatic xenograft model bearing both PANC-1 (FR+) and MIA PaCa-2 (FR-) tumors, the mice were imaged with [¹²⁴I]L4 (6.7-9.3 MBq in 200 µL PBS) at 24 h post-injection, when the tumor of both sides reached a diameter of 4-6 mm.

The establishment of the tumor in the orthotopic pancreatic tumor models was confirmed at 13 days post-inoculation of PANC-1/Luc+ cells into the pancreas tail with bioluminescence imaging using D-luciferin (60 mg/kg). The next day, [¹⁸F]FDG-PET/CT (FDG: fluorodeoxyglucose) imaging was performed at 1 h post-injection (7.4 MBq/mouse) for 20 min. Two days after [¹⁸F]FDG imaging, [¹²⁴I]L4 (6.3-9.3 MBq in 200 µL PBS) was administered into the tail vein of the same mouse and PET/CT images were acquired at 1, 4, and 24 h post-injection.

Right after PET imaging at 24 h post-injection, Cerenkov luminescence images were acquired using IVIS Lumina-XR (PerkinElmer, Waltham, USA) under open-abdominal conditions to confirm visually the exact location of the radioactive signal. The Cerenkov luminescence signal was obtained in a bioluminescence channel without excitation and with the following conditions: open emission filter; exposure time, 5 min; binning, medium: 8; field of view, D (22.5 × 22.5 cm); f/stop, 1. The images were quantitatively analyzed by the Living Image software.

Bioluminescence images were also acquired 10 min after the intraperitoneal injection of D-luciferin (60 mg/kg) to confirm the consistency between the PET, Cerenkov luminescence, and bioluminescence signals of the orthotopic pancreatic tumor. The bioluminescence imaging conditions were as follows: open emission filter; exposure time, 10 s; binning, medium 8; field of view, D (22.5 × 22.5 cm); f/stop, 1.

After bioluminescence imaging, the mouse was immediately sacrificed and the tissues were resected for *ex vivo* bioluminescence imaging under the same conditions. I-¹³¹ was also used to acquire single-photon emission computed tomography (SPECT) images with multi-pinhole collimators. At 24 h post-injection, the SPECT images were acquired with a four-head NanoSPECT/CT scanner (Mediso, Budapest, Hungary) for 100 min using orthotopic pancreatic tumor model and NuclineTM software. The imaging parameters were as follows: isotropic voxel sizes, 100 µm; ten-pinhole mouse brain apertures; pinhole diameter, 1.0 mm; 24 projections; axial field of view, 20.9 mm. The acquired SPECT images were reconstructed with InterView^{™} Fusion software, while the animals were anesthetized with isoflurane (1-2% in O₂) during the scan to obtain CT images, which were automatically co-registered with the SPECT images.

### Results

A high signal-to-noise ratio facilitates disease diagnosis and minimizes the possibility of misreading in all imaging modes. In theory, the signal-to-noise ratio can be increased simply by increasing the signal in the target while reducing it in the background via the selective delivery of the imaging agent to the target. For tumor imaging, various nanoparticles of 20-200 nm have shown good tumor uptake due to their enhanced permeability and retention (EPR) effect in the poor tumor neovasculature. In addition to the passive EPR effect, the targeting ability of nanoparticles has been further increased by attaching tumor-specific biomolecules on the nanoparticle surface. However, although tumor signals could be increased by utilizing these strategies, the accompanying uptake of the imaging agent in surrounding organs creates unavoidable background noise. In particular, only a small portion of the injected nanoparticles was delivered to the tumor, while most of them were trapped in the liver and spleen. A recent meta-analysis also showed that less than 1% of the injected nanoparticles reach the tumor, whereas 99% of them are taken up and/or eliminated by the mononuclear phagocytic and renal system. The liver sequesters up to 70% of the injected nanomaterials.

The inventors continued their research to elucidate the mechanisms underlying the differential clearance of radioactive tracers from tumors and other organs.

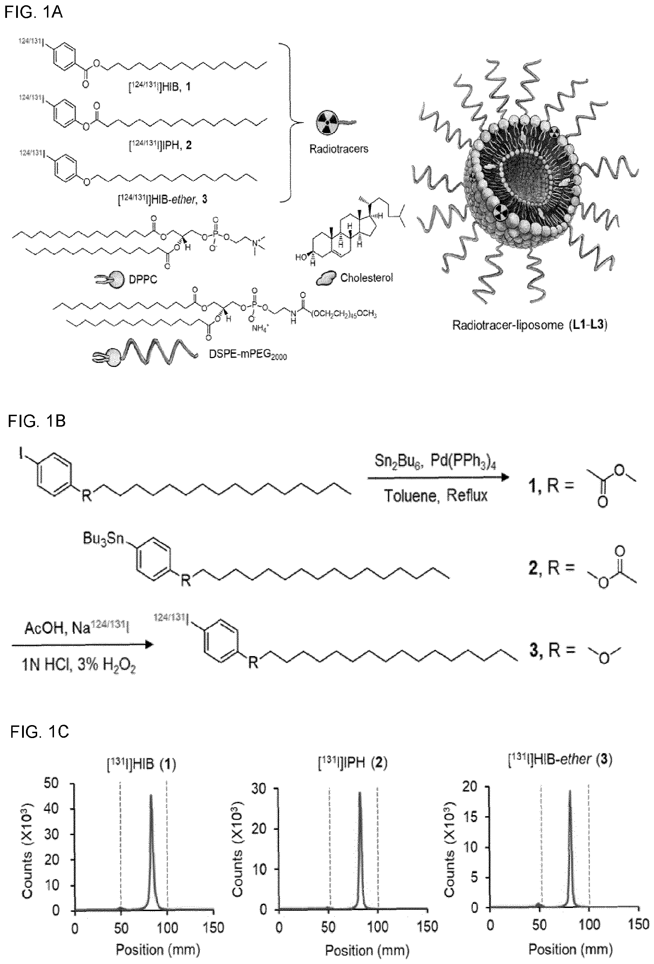

We first tried to further increase the selective tumor uptake by systematically changing the ratio of the liposomal components 1,2-dipalmitoyl-sn-glycero-3-phosphocholine (DPPC), 1,2-dihexadecanoyl-sn-glycero-3-phospho-(1'-rac-glycerol) (DPPG), cholesterol, and 1,2-distearoyl-*sn*-glycero-3-phosphoethanolamine-*N-*[methoxy(polyethylene glycol)-2000] (DSPE-mPEG₂₀₀₀) (FIG. 1A).

Comparison of the percentage of injected dose per gram (%ID/g) in the tumor indicated that the liposomal component ratio significantly affected the tumor uptake, which ranged from 0.11 ± 0.08 to 3.97 ± 0.27 %ID/g. Especially when anionic DPPG was replaced by neutral DPPC without changing the ratio of cholesterol and DSPE-mPEG₂₀₀₀, the tumor uptake increased from 1.32 ± 0.27 to 3.97 ± 0.27 %ID/g (Composition 6). In contrast, the liver and spleen uptake varied to a smaller extent regardless of the liposomal component ratio.

These results suggest that the optimization of the liposomes' composition can increase the tumor uptakes but does not significantly affect activity clearance from the MPS organs.

The next possibility was the radiotracer [¹²⁴I]HIB itself. Lipophilic [¹²⁴I]HIB is expected to be cleared by the hepatobiliary tract because of its high lipophilicity (log P = 2.19 ± 0.04). However, after an initial high uptake in the liver, most of the radioactivity was excreted from the body in the urine rather than feces, strongly suggesting that [¹²⁴I]HIB was degraded in the liver and that the hydrophilic metabolites re-entered the bloodstream and were excreted by the renal system. We paid attention to the ester linkage of [¹²⁴I]HIB that can be metabolized in vivo to give hydrophilic 4-[¹²⁴I]iodobenzoic acid and lipophilic 1-hexadecanol.

To verify our hypothesis by direct comparison, we synthesized two radiotracers, 4-[^{124/131}I]iodophenyl heptadecanoate ([^{124/131}I]IPH, 2) and 1-(hexadecyloxy)-4-[^{124/131}I]iodobenzene ([^{124/131}I]HIB-ether, 3) (FIG. 1A). Similar to 1, radiotracer 2 contained an ester bond between the 4-iodophenyl and hexadecyl alkyl groups but in the reverse direction. In contrast, the 4-iodobenzene and hexadecyl alkyl groups in 3 were linked via an ether bond.

For the synthesis of [^{124/131}I]IPH and [^{124/131}I]HIB-ether, p-iodophenol reacted with heptadecanoic acid and 1-iodohexadecane to give 4-iodophenyl heptadecanoate and 1-(hexadecyloxy)-4-iodobenzene, respectively. These non-radioactive compounds were then converted to their 4-(tributylstannyl)phenyl precursors for radioiodination upon reaction with hexabutylditin and a catalytic amount of Pd(PPh₃)₄ (FIG. 1B).

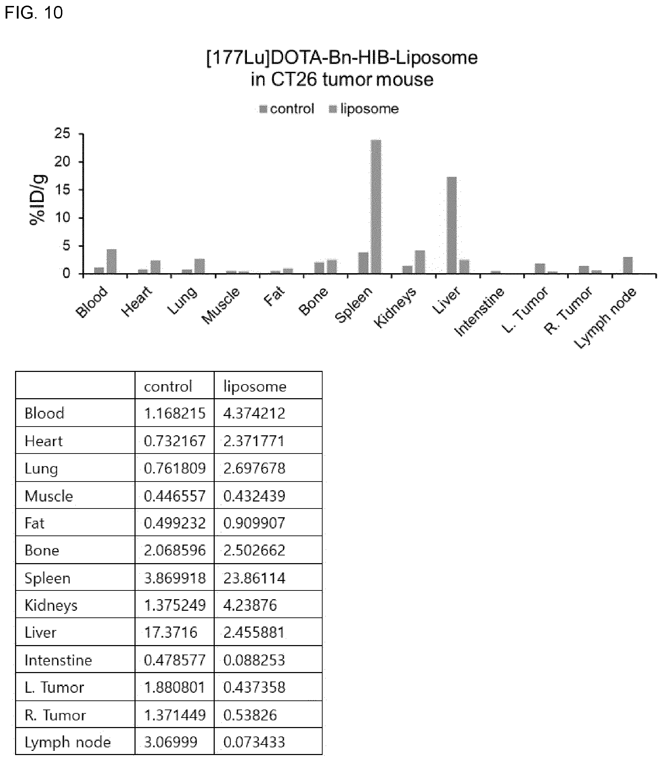

Radioiodination was then performed by replacing the tributylstannyl groups of the synthesized precursors with [¹³¹I]Nal or [¹²⁴I]Nal (18.5-185 MBq) in the presence of acetic acid and 3% H₂O₂ (FIG. 1B). The respective radiolabeling yield and radiochemical purity for both [^{124/131}I]IPH and [^{124/131}I]HIB-ether after purification were 51-69% and >98%, respectively (FIG. 10).

The prepared radiotracers were then incorporated into an optimized PEGylated liposome by slightly modifying an existing method (FIG. 1A). In particular, each radiotracer was added to a solution of DPPC, cholesterol, and DSPE-mPEG₂₀₀₀ in chloroform at a molar ratio of 16:1:7. The well-mixed solution was then slowly dried to form a thin lipid film, hydrated with PBS at 50 °C, and extruded sequentially through membrane filters with pore sizes of 1000, 400, 200, and 100 nm. Before use, the synthesized liposomes were purified and concentrated by centrifugal filtration (YM-100, Millipore, St. Louis, MO, USA).

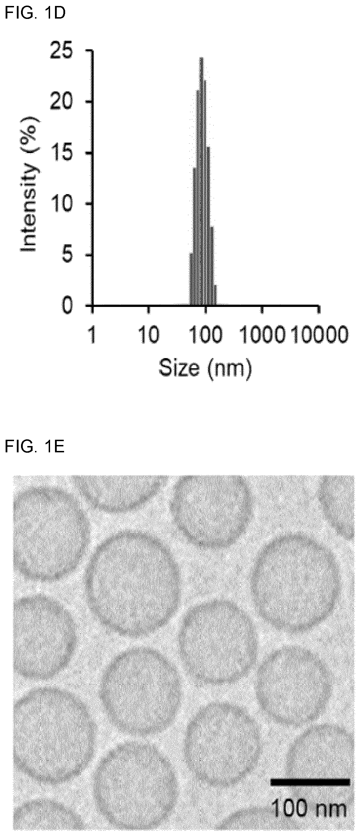

The average tracer incorporation yields of [¹²⁴I]HIB-liposome (L1), [¹²⁴I]IPH-liposome (L2), and [¹²⁴I]HIB-ether-liposome (L3) ranged between 46.5% and 58.8%. The hydrodynamic size and zeta potential of [¹³¹I]L1 were determined at 112.4 ± 37 nm and -31.4 ± 5.1 mV, respectively, using a dynamic light scattering (DLS) size analyzer (FIG. 1D). A similar size (103.4 ± 0.5 nm) was also determined by nanoparticle tracking analysis. Negative staining cryo-TEM image of three liposomes exhibited a globular shape in the diameter of ~100 nm, which is in full agreement with the DLS determinations (FIG. 1E).

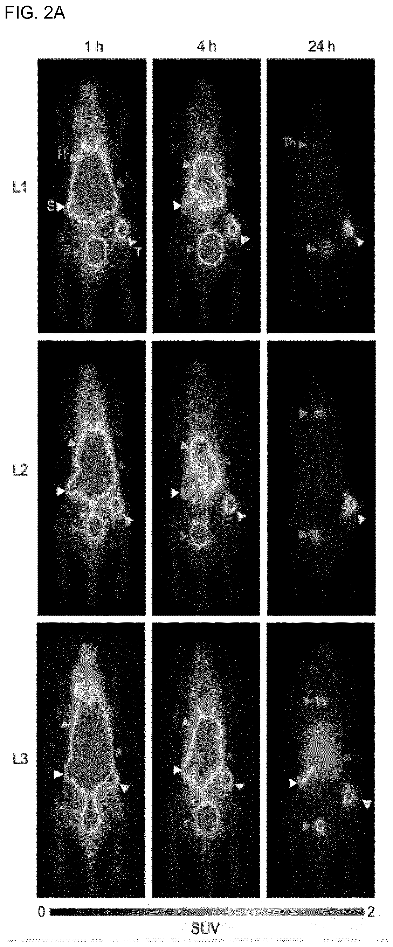

The tumor uptake and body clearance of [¹²⁴I]L1-L3 were then compared in CT26 tumor-bearing BALB/c mice using animal PET images recorded at 1, 4, and 24 h post-injection (FIG. 2A).

Although L1 and L2 with ester-linked radiotracers showed similar high uptake and clearance patterns, liposome L3, which was loaded with an ether-linked radiotracer, showed a different in vivo behavior.

The maximum intensity projection (MIP)-PET images of L1 and L2 at 1 h post-injection showed high uptakes in the abdominal lesion and some activity in the tumor. However, after 4 h, the abdominal radioactivity decreased significantly, while the urine radioactivity in the bladder continued to increase. At that time point, tumors were clearly visualized in high contrast, as the background radioactivity around the tumors cleared faster than the tumor radioactivity. At 24 h post-injection, only tumors were clearly visualized with an unusually low background radioactivity in the abdominal region.

However, in the PET images of [¹²⁴I]HIB-ether-liposomes (L3), the clearance from the liver and spleen was much slower than those in the other liposomes (FIG. 2A, bottom). Up to 4 h, no dramatic clearance was observed and the radioactivity of L3 in the liver and spleen remained high. At 24 h, more radioactivity was excreted from the abdominal region, being the tumor more clearly visualized. However, compared with L1 and L2, significant radioactivity was still observed in the MPS organs in L3.

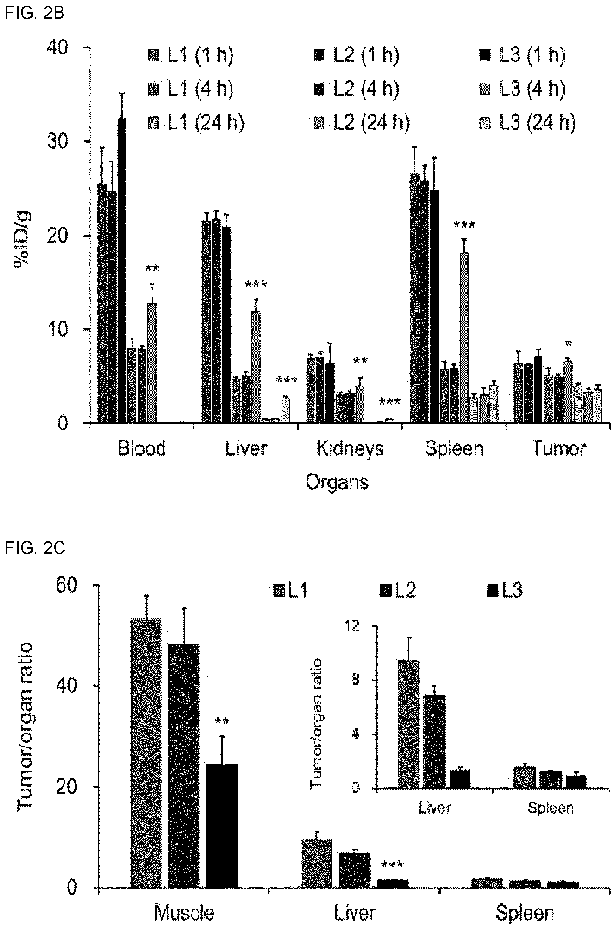

The behavior of [¹³¹I]L1-L3 was further analyzed by *in vivo* biodistribution studies on the same CT26 tumor models (FIG. 2b-c, S15a, and Table S2). The biodistribution data were in good agreement with the PET imaging results.

At 1 h post-injection, the uptake of L1-L3 in all organs was comparable within the error range, suggesting that the initial biodistribution patterns of radiotracers were governed solely by the liposomes and that tracers moved together with the liposomes without dissociation.

However, at 4 h, distinctive uptake patterns were observed between the ester- and ether-linked radiotracers in the liver and spleen. Specifically, more than 75% of the radioactivity of L1 and L2 was cleared from the liver within 3 h, whereas a decrease of <44% was observed in the radioactivity of L3. After 24 h, the radioactivities of L1 and L2 were further reduced to <0.5 %ID/g, and only ~2% of their radioactivity at 1 h post-injection was detected in the liver. In contrast, the radioactivity of L3 in the liver was more than six times higher. A similar clearance pattern was observed in the spleen. However, no significant difference was observed in the clearance patterns of L1, L2, and L3 in the tumor, while the clearance rate was much slower for all liposomes. Less than 50% of the radioactivity at 1 h was cleared at 24 h post-injection. More than 1% of the injected radioactivity still remained in the tumor at 24 h post-injection, which accounts for ~20% of total body activity in L1 and L2. The tumor-to-muscle uptake ratios of L1 and L2 at 24 h post-injection were ~50, which was two-fold higher than that of L3 (FIG. 2C). The tumor-to-liver ratios of ester-linked radiotracers (L1 and L2) were more than five times higher compared to ether-linked radiotracer (L3).

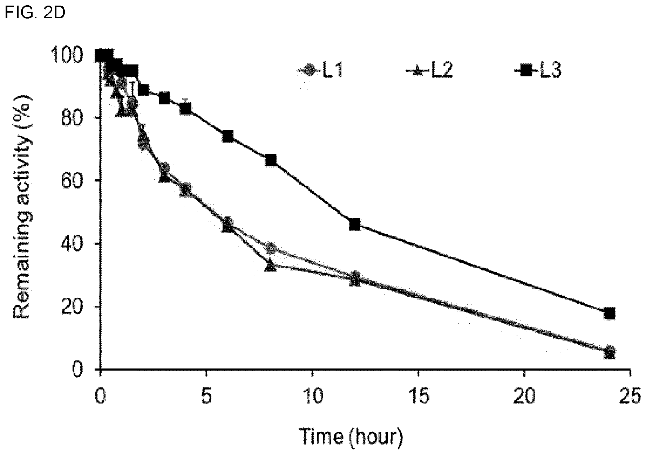

The faster body clearance ester-linked radiotracers was also clearly shown by examining the remaining radioactivity in tumor-bearing mice over time (24 h) (FIG. 2D). L1 and L2 had very similar body clearance patterns. At 4 h post-injection, ~43% of the initial L1 and L2 radioactivities were excreted from the body, whereas only 17% of the injected radioactivity was cleared in L3. At 24 h, approximately three times higher radioactivity was found in L3-treated mice at 24 h post-injection compared to L1- and L2-treated mice.

All of the above PET imaging and biodistribution data strongly imply that some metabolic pathways related to a specific bond (ester or ether) are closely involved in this differential clearance pattern, and the metabolic activity is highly dependent on tissue and organs.

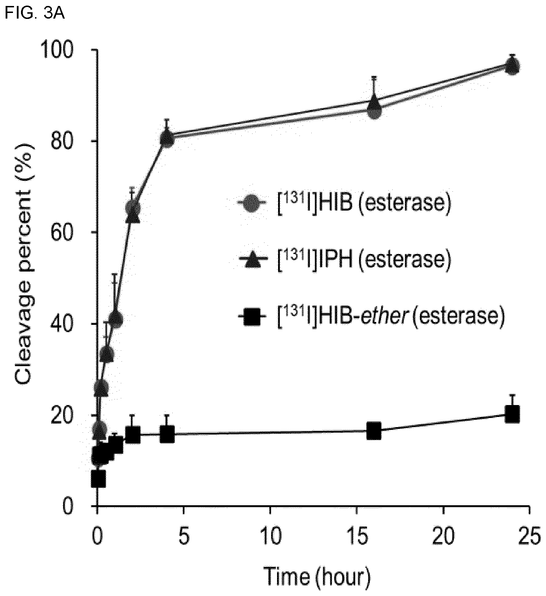

With these hypotheses in mind, we performed systematic mechanistic studies. First, we carried out an esterase enzymatic assay. All three radiotracers, [¹³¹I]HIB, [¹³¹I]IPH, and [¹³¹I]HIB-ether, were incubated separately with esterase (Sigma Aldrich, St. Louis, MO, USA) at 37 °C in a 10% DMSO/PBS mixture, and the progress of the reactions was monitored for 24 h by radio-TLC (FIG. 3A).

As expected, rapid bond cleavage was observed in the two ester-linked radiotracers, [¹³¹I]HIB and [¹³¹I]IPH. Within 4 h, >80% of [¹³¹I]HIB and [¹³¹I]IPH degraded, and the cleavage percentage increased to >96% at 24 h. However, the ether-linked radiotracer, [¹³¹I]HIB-ether showed only 15.9% and 20.3% cleavage at 4 and 24 h, respectively, which was five times lower than the cleavage achieved in [¹³¹I]HIB and [¹³¹I]IPH. Taken together, we showed that esterase hydrolyzed effectively the ester bonds of [¹³¹I]HIB and [¹³¹I]IPH, but did not affect the ether bond in [¹³¹I]HIB-ether.

To further validate our results, we repeated the same cleavage experiment using the mouse liver S9 fraction and microsome. Both have been widely used in hepatic metabolism studies due to high content in various enzymes. The liver S9 fraction contains microsomal and cytosolic fractions.

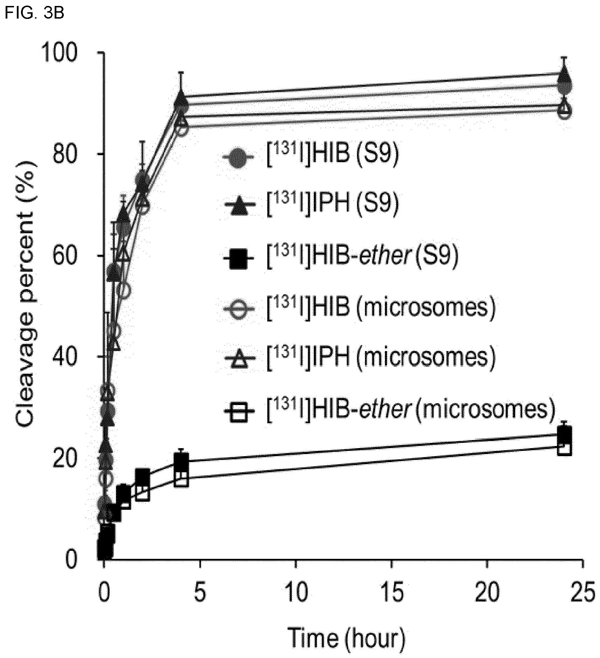

The general cleavage patterns of the three radiotracers were very similar to those of esterase (FIG. 3B). The two ester-linked radiotracers ([¹³¹I]HIB and [¹³¹I]IPH) showed much faster cleavage rates than the ether-linked radiotracer ([¹³¹I]HIB-ether). Within 4 h of incubation, the cleavage percentages of [¹³¹I]HIB and [¹³¹I]IPH increased to 89.7% and 91.3% in the S9 fraction and 85.3 % and 87.3% in the microsome, respectively. By contrast, [¹³¹I]HIB-ether showed only 19.3% and 16.0% cleavage in S9 and microsome at 4 h, which increased slightly further at 24 h. Interestingly, all radiotracers showed 5-20% higher cleavage rates in S9 than in the microsome, suggesting that most enzymes involved in the bond cleavage of the three radiotracers originated from the microsome and not from the cytosol. These data were in line with the fact that carboxylesterases are mainly found on the lumen side of the endoplasmic reticulum, the major component of the microsome, while their abundance in the cytosol is low.

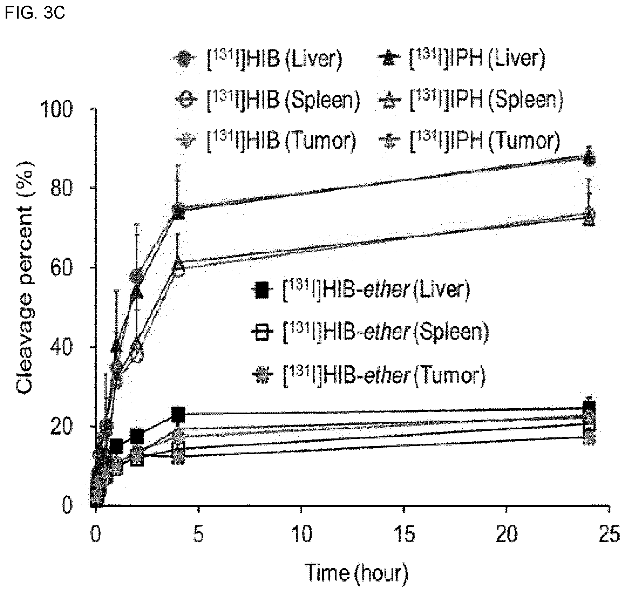

Next, the present inventors performed *Ex vivo* studies to compare the activities of esterase in different organs. The cleavage rates of the three radiotracers were measured in freshly prepared liver, spleen, and CT26 tumor homogenates. As expected, the cleavage rates in the liver homogenate were very similar to those in S9 and microsome (FIG. 3C). The ester-linked radiotracers [¹³¹I]HIB and [¹³¹I]IPH showed much faster cleavage (-75% [4 h], -88% [24 h]) than ether-linked [¹³¹I]HIB-ether (23% [4 h], 25% [24 h]). The same cleavage pattern was also observed in the spleen homogenate, although the cleavage rates of [¹³¹I]HIB and [¹³¹I]IPH at 4 h and 24 h were -18% lower than those in the liver. Similar to the liver homogenate, the cleavage percentage of [¹³¹I]HIB-ether in the spleen was significantly lower than that of the ester-linked radiotracers. In contrast, the cleavage rates of all radiotracers in the tumor were comparable, <20% at 4 h and <23% at 24 h, strongly suggesting that the esterase activity in the tumor was much lower than in the liver and spleen.

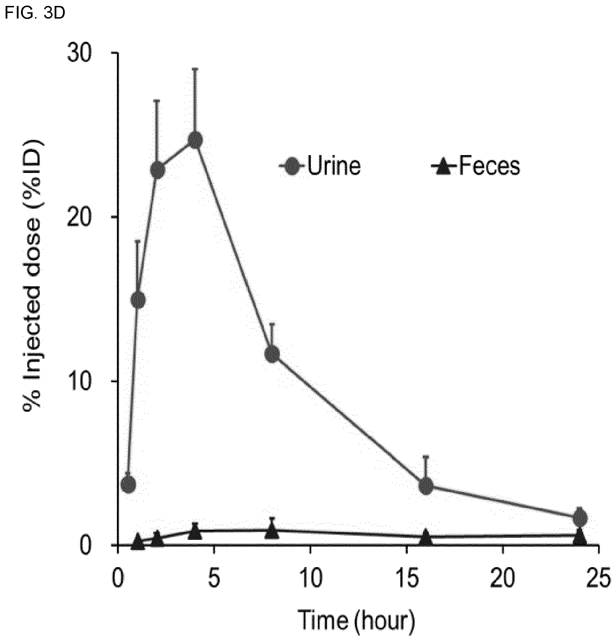

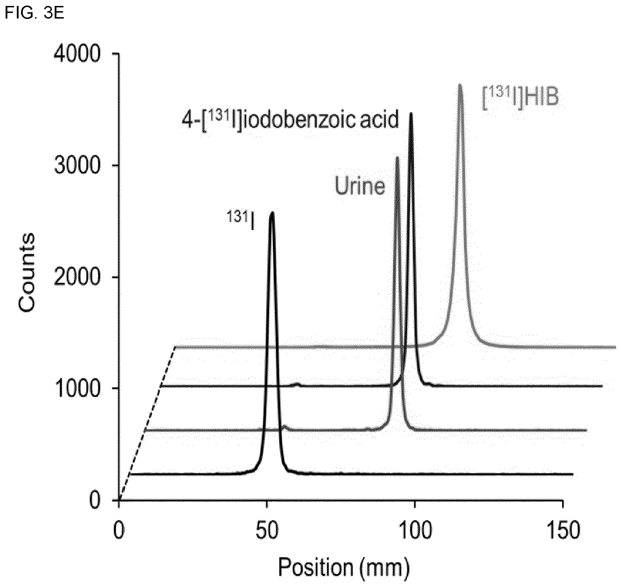

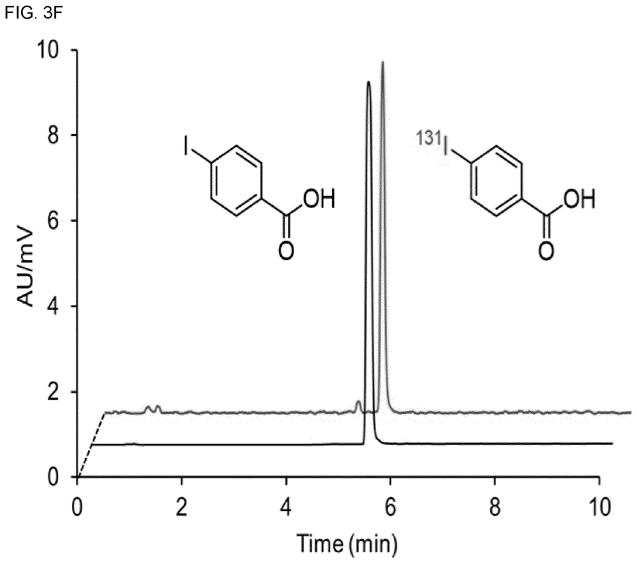

Finally, we directly analyzed the metabolites of [¹³¹I]HIB in urine and fecal samples. Most radioactivity was excreted in the urine rather than in the feces. The radioactivity in the feces was less than 5% of that in urine (FIG. 3D). Radio-TLC analysis of urine samples collected at 3 h post-injection indicated the formation of 4-[¹³¹I]iodobenzoic acid, which can be generated from the ester bond cleavage in [¹³¹I]HIB by esterase (FIG. 3E). The tracer peak at the origin of theTLC plate was assigned to free ¹³¹I ions. The [¹³¹I]HIB metabolites in the urine sample were further confirmed by HPLC using 4-iodobenzoic acid as the standard compound (FIG. 3F). The radio-HPLC peak in the urine sample matched exactly with the UV peak of 4-iodobenzoic acid.

These results clearly showed that lipophilic [¹³¹I]HIB loaded onto the liposome was metabolized to hydrophilic 4-[¹³¹I]iodobenzoic acid by esterase mainly in the liver and spleen and was then excreted via the renal system rather than the hepatobiliary tract.

After determining the mechanism underlying the differential clearance of esterase-labile radiotracers, we decided to utilize this finding in addressing unmet clinical needs. Since this differential clearance mechanism might be a general phenomenon in different cancer types, we considered that this finding could be used to develop the imaging strategy of early cancer detection with significantly improved contrast.

In particular, the detection of tumors in the abdominal region can benefit from this imaging strategy. For this analysis, we selected pancreatic cancer. Pancreatic cancer has the highest mortality rate among all major cancers. Its median survival after diagnosis is only six months. Moreover, the 5-year survival rate has remained unimproved at -10% for the last 40 years. Because the pancreas is located deep in the abdomen and is surrounded by other major organs, such as the liver, kidney, stomach, and intestine, pancreatic cancer less than 1 cm in size is highly difficult to diagnose even with modern medical imaging technologies such as ultrasound, CT, and MRI. Although FDG-PET is highly effective for the early detection of various types of cancer, its sensitivity and specificity are insufficient for pancreatic cancer.

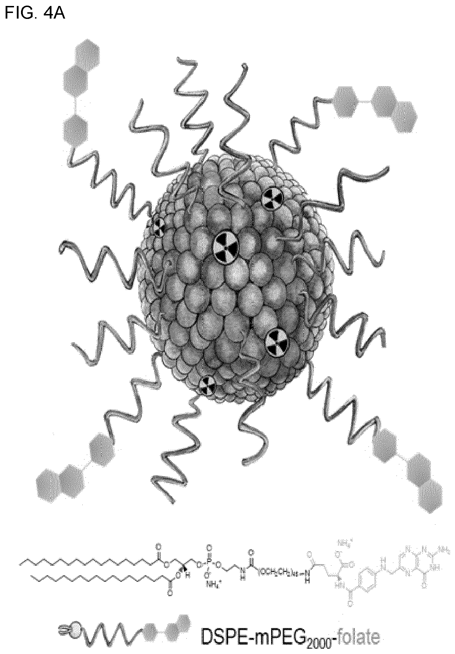

Here, in order to increase tumor targeting efficiency for pancreatic cancer, we optimized the liposome surface of [¹²⁴I]HIB-liposome (L1) with folate. Folate, also known as vitamin B9, is essential for cell proliferation and is transported within the cells through the folate receptor (FR) on the cell membrane. Given the overexpression of FR in several types of cancer, including pancreatic cancer, these receptors constitute an appropriate target for tumor diagnosis and therapy for decades. The optimized liposomal composition of folate-[¹²⁴I]HIB-liposome (L4) was 16:1:5:2 between DPPC, cholesterol, DSPE-mPEG₂₀₀₀, and DSPE-mPEG₂₀₀₀-folate, where DSPE-mPEG₂₀₀₀-folate is 1,2-distearoyl-*sn*-glycero-3-phosphoethanolamine-*N-*[folate(polyethylene glycol)-2000] (FIG. 4A). The hydrodynamic size of ~100 nm and globular shape of L4 was confirmed by DLS and cryo-TEM, respectively (Figure S17b & c). The folate-[¹³¹I]HIB-liposome (L4) showed an excellent liposomal integrity, as well as, radiochemical stability in PBS up to two weeks.

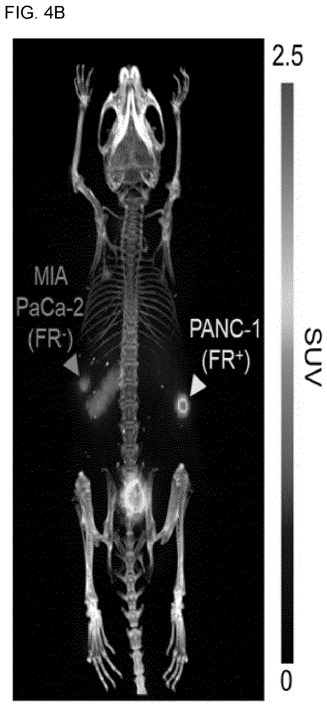

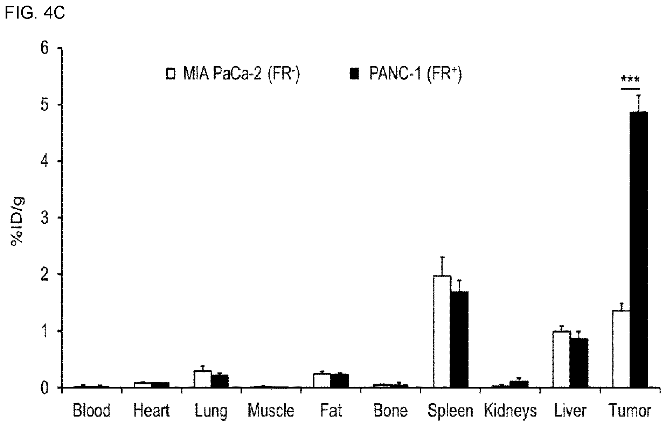

The targeting efficiency of [¹²⁴I]L4 was first evaluated through animal PET imaging in tumor xenograft models bearing the human pancreatic cancer cell lines PANC-1 (FR overexpressing, FR+) and MIA PaCa-2 (FR deficient, FR-). As expected, PANC-1 tumor on the right flank showed higher uptake than MIA PaCa-2 on the left flank at 24 h post-injection (FIG.4B). Similar to the PET image of L1 in CT26 tumor models (FIG. 2A), the radioactivity was rapidly cleared from the liver and spleen through the urinary tract. A separate biodistribution study at the same time point showed that the tumor uptake of PANC-1 was 3.6 times higher than that of MIA PaCa-2, showing that the higher tumor uptake in PANC-1 (FR+) was mediated by the active targeting of folate on the surface of [¹²⁴I]L4 (FIG. 4C). The tumor (PANC-1)-to-organ ratios of the liver, spleen, and kidney were high (T/L 5.6, T/S 2.9, and T/K 43) due to the fast clearance from the background, while the tumor-to-blood and tumor-to-muscle ratios were unexceptionally high (T/B 212 and T/M 270).

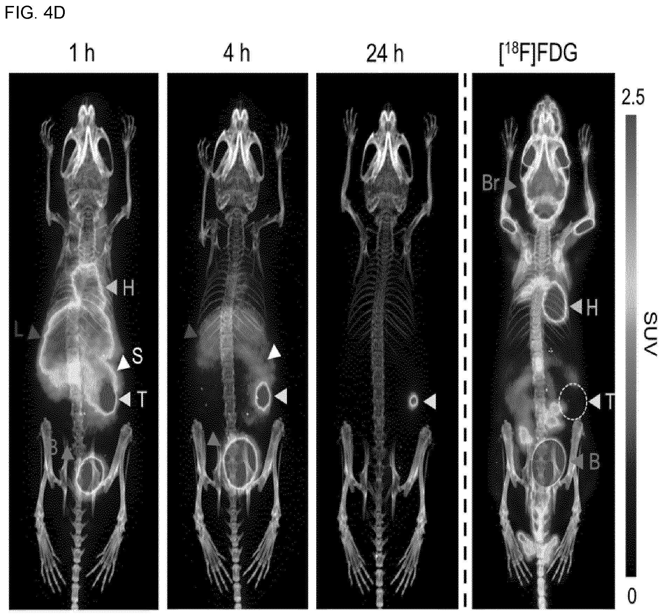

Prompted by the promising results in the xenograft tumor models, we subsequently used orthotopic pancreatic cancer models. PANC-1 cells transfected with luciferase (PANC-1/Luc+) were injected orthotopically in the pancreatic tail of BALB/c nude mice. At 2 weeks post-inoculation, L4 was injected via the tail vein, and the mice were imaged by PET imaging (FIG. 4D).

At 1 h post-injection, the hot spots around the pancreatic lesion were clearly recognized, but high radioactivities were also found in the abdominal region, especially in the liver and spleen. The highest radioactivity was found in the bladder, indicating the fast renal clearance of the hydrophilic [¹²⁴I]HIB metabolite. At 4 h post-injection, most of the abdominal radioactivity was excreted in the bladder, and only the pancreatic tumor was clearly visualized, while at 24 h, the radioactivity in all organs was significantly reduced and only the tumor could be detected with practically no background signals.

However, when the same mouse was imaged by [¹⁸F]FDG-PET, the tumor could barely be detected and high uptakes were observed in the other glucose-avid organs (FIG. 4D).

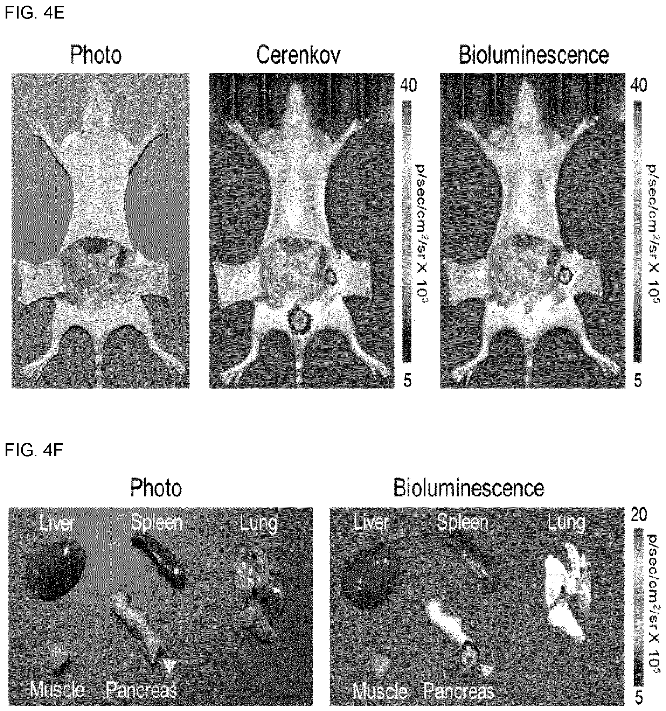

The mouse abdominal organs were exposed immediately after 24 h PET imaging and imaged by Cerenkov imaging to confirm that the detected focal hot spot originated from the orthotopically inoculated pancreatic tumor. Radioisotope I-124 emits relatively strong Cerenkov radiation that can be detected by a sensitive CCD camera within seconds. Here, we detected two focal signals in the Cerenkov image (FIG. 4E, middle); one in the pancreas and one in the bladder. The anesthetized mouse was further injected with D-luciferin intraperitoneally for bioluminescence imaging (FIG. 4E, right). A single focal spot was detected at exactly the same position as the hot spot of Cerenkov imaging, confirming that the strong PET signal at 24 h post-injection was from the pancreas and originated specifically from orthotopically inoculated PANC-1/Luc+ cells. These results were further confirmed by ex vivo bioluminescence imaging of the excised organs, where only a ~2 mm tumor was clearly detected in the pancreas tail, but no detectable signals were observed in all other organs, including normal pancreas tissue (FIG. 4F).

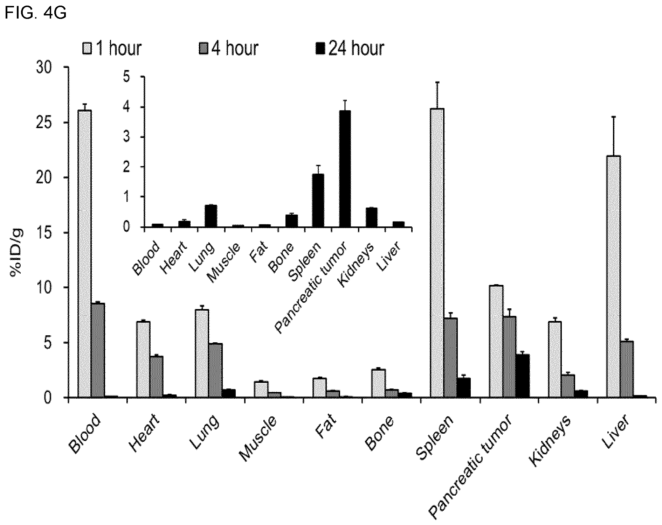

The biodistribution data of folate-[¹³¹I]HIB-liposome ([¹³¹I]L4) in orthotopic pancreatic tumor models matched well with PET imaging. At an early time point (1 h), the highest uptake was found in the spleen, followed by the blood and the liver (FIG. 4G). The radioactivity in the pancreatic tumor was the fourth highest. The muscle showed the lowest uptake among the collected organs. At 4 h post-injection, the uptake in the tumor was the second highest after that in the blood. During the first 3 h, while only 27% of the radioactivity at 1 h cleared from the tumor, 67%, 73%, and 77% of the 1 h radioactivity cleared from the blood, spleen, and liver, respectively. At 24 h, most activities from all organs except the tumor and spleen cleared to leave less than 1%ID/g (FIG. 4G, insert). At 24 h, 38% of the 1 h radioactivity remained in the tumor, while more than 93% of the 1 h radioactivity had cleared from the spleen and liver. The tumor-to-blood, tumor-to-muscle, tumor-to-liver, tumor-to-kidney, and tumor-to-spleen ratios were calculated to be 49, 65, 24, 6.3, and 2.2, respectively, at 24 h post-injection (Table S3), which are exceptionally high for tumor imaging utilizing liposomal imaging probes.

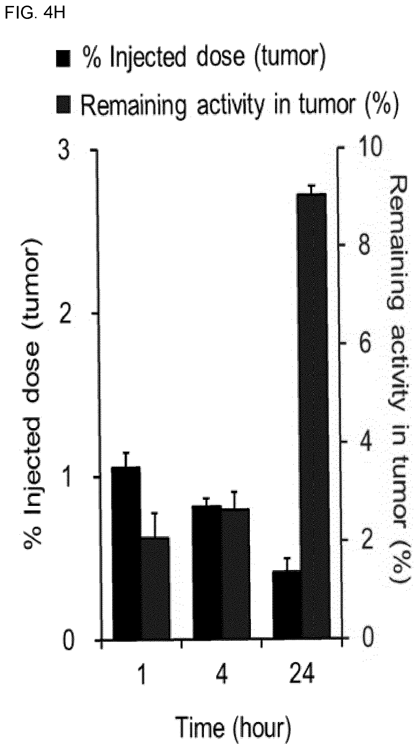

Although the tumor uptake ratios of the injected radioactivity slowly decreased from 1.1% to 0.8% and 0.4% at 1, 4, and 24 h post-injection, the tumor radioactivity as a percentage of total body radioactivity increased rapidly from 2.0% to 2.6% and 9.0% at 1, 4, and 24 h, respectively (FIG. 4H). When considering that the orthotopic tumor weight was only 0.03% of the total body weight, the localization of 9.0% radioactivity in the tumor is an exceptionally high value, to our best knowledge.

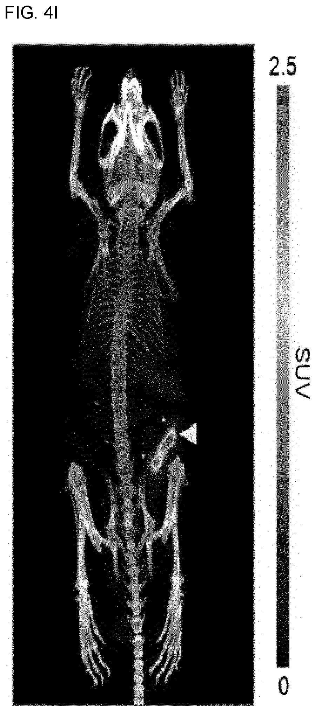

When considering the higher accessibility of ¹³¹I (or ¹³¹I) radioisotope and single-photon emission computed tomography (SPECT) scanner compared with I-¹²⁴ and PET imaging scanners, ¹³¹I-labeled folate-[¹³¹I]HIB-liposome ([¹³¹I]L4) would be an excellent alternative in future clinical applications. When an orthotopic pancreatic tumor model was imaged by SPECT after the injection of [¹³¹I]L4, a highly comparable clean tumor image was obtained with very minimal background at 24 h, similar to PET images (FIG. 4I).

### II. Example 2: Synthesis of DOTA-Benzene-HIB and its utilization

### 1. Synthesis of experimental substances

### 1) Boc-4-aminobenzoic acid

### (1) Synthesis of Boc-4-aminobenzoic acid

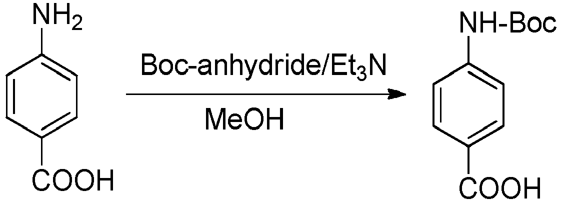

p-Aminobenzoic acid (1.5 g, 1 eq) was dissolved in 100 ml of methanol, and boc-anhydride (4.5 g, 2 eq) and Et₃N (5 ml, 2eq) were added to the solution at 0°C. The reaction solution was stirred for 16 h at room temperature. Then, the solution was concentrated and extracted with hexane and saturated aqueous sodium bicarbonate. The water phase was then acidified with 10% aqueous citric acid to pH 4 to obtain white solid (1.321 g, yield- 50.2%).

### (2) Synthesis of hexadecyl 4-((tert-butoxycarbonyl)amino)benzoate

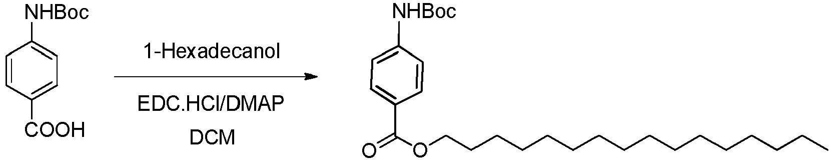

Boc- 4-amino benzoic acid (1.32 g, 1 eq) was dissolved in 100 ml of DCM, and 1-hexadecanol (1.473 g, 1.1 eq), EDC. HCl (1.261g, 1.2eq) and DMAP (67.5 mg 0.1 eq) were added to the solution at 0 °C. The reaction solution was stirred for 16 h at room temperature. Then, the solution was concentrated and extracted with DCM. Purified by silica gel column chromatography obtains white color solid (1.39 g, 51.4%).

### (3) Synthesis of hexadecyl 4-aminobenzoate

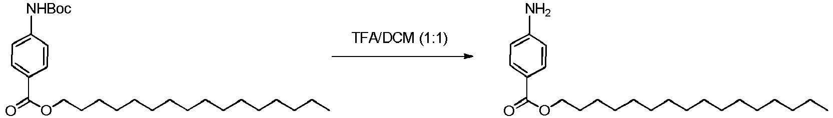

Hexadecyl 4-((tert-butoxycarbonyl)amino)benzoate(1.20 g, 1 eq) was dissolved in 5 ml of DCM, and 5 ml of TFA were added to the solution at 0 °C. The reaction solution was stirred for 5 h at room temperature. Then, the solution was concentrated and extracted with DCM. Washed with NaHCOs solution. White color solid (0.88 g, 93.7%).

### (4) Synthesis of DOTA-Benzene-HIB

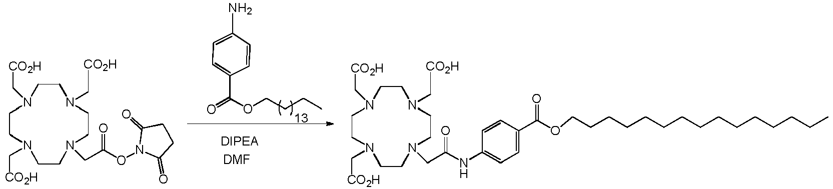

Hexadecyl 4-aminobenzoate (2.37 mg, 1 eq) was dissolved in 0.2 ml of DMF, then DIPEA (11µl, 10 eq). Then add DOTA-NHS ester (5.5 mg 1.1 eq) in 0.8 ml DMF. The reaction solution was stirred for 12 h at room temperature. Purification done by using waters HPLC isolated 250 µg- white solid.

### (5) Synthesis of ⁶⁴Cu-DOTA-Benzene-HIB

Isotope Cu-64 was labeled on the synthesized DOTA-Benzene-HIB, and the labeling rate was confirmed by Radio-TLC, and the labeling rate was 96.06%.

### (6) Synthesis of ⁶⁴Cu-DOTA-Benzene-HIB-Liposome

800 µL of 0.5mM DPPC, 350 µL of DSPE-PEG₂₀₀₀, and 50 µL of cholesterol were added to a 100 mL round flask. After lightly stirring them for about 5 seconds (using a chloroform solvent), a thin film was formed using an evaporator. After concentration under reduced pressure was completed, 1 mL of PBS was added to the round flask. Hydration was performed in a heating bath at 55°C. After hydration, the PBS solution was extracted with a glass syringe, and extrusion was performed 19 times each at 400 nm and 100 nm in size at 50 °C.

### 2. In vivo experiment of ⁶⁴Cu-DOTA-Benzene-HIB

### (1) Research on in vivo excretion

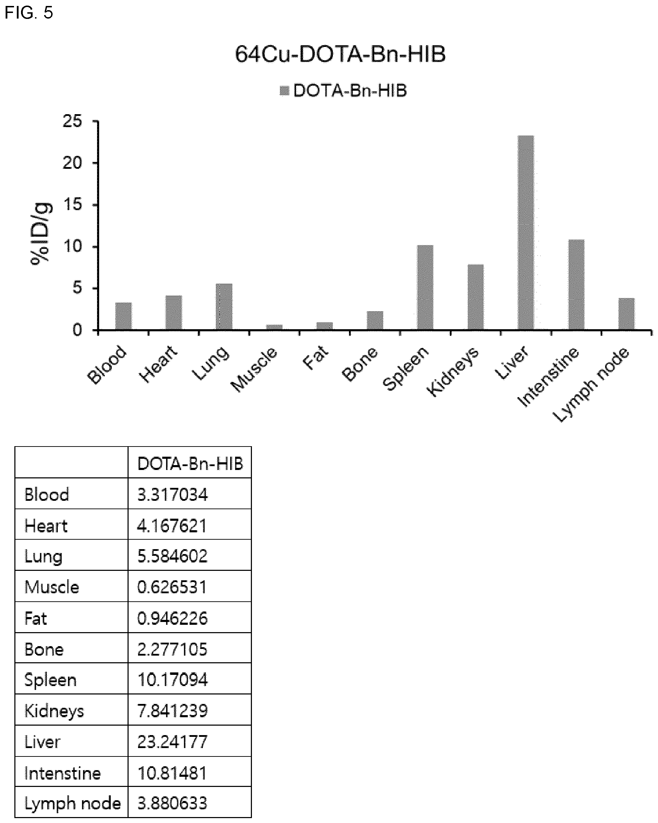

An experiment was conducted to determine the in vivo excretion rate of ⁶⁴Cu-DOTA-Benzene-HIB. ⁶⁴Cu-DOTA-Benzene-HIB was injected into mice and biodistribution was performed 7 hours later. The biodistribution results showed low uptake in the blood and heart, and high uptake in the liver, kidney, and intestine, confirming a typical in vivo excretion pattern (FIG. 5).

### (2) PET/CT imaging

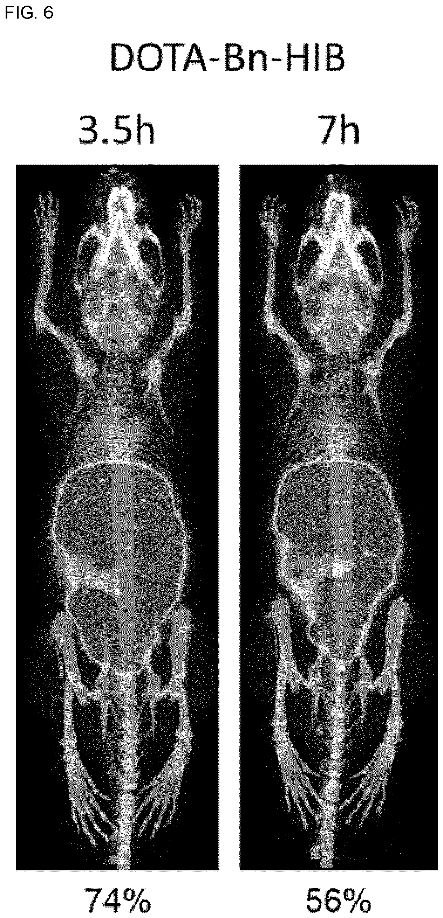

⁶⁴Cu-DOTA-Benzene-HIB was injected, and PET/CT analysis was performed for 3.5 and 7 hours. The PET/CT analysis showed the in vivo residual activity of 74% in the mouse after 3.5 hours, and the residual activity of 56% in the mouse after 7 hours. A high uptake was shown in the liver, kidney and intestine, which were typical in vivo excretion patterns confirmed by imaging. Since the heart signal was weaker after 7 hours than after 3.5 hours, it was confirmed that ⁶⁴Cu-DOTA-Benzene-HIB was still circulating in the body. The same in vivo excretion patterns as the results of biodistribution were confirmed (FIG. 6).

### 3. ⁶⁴Cu-DOTA-Benzene-HIB-Liposome tumor uptake

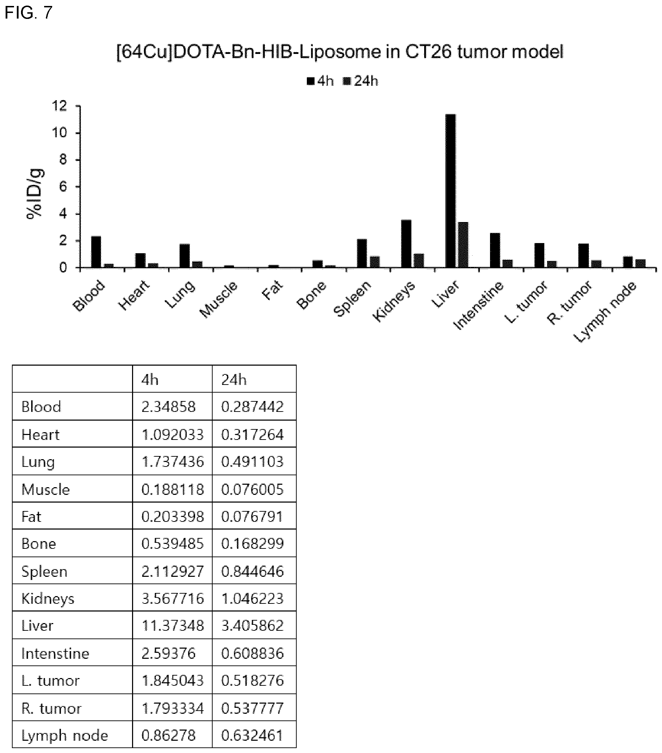

There was a CT26 tumor on both sides of the mouse, and tumor uptake was evaluated using ⁶⁴Cu-DOTA-Benzene-HIB-Liposome. The tumor uptake of mice 4 hours after injection of ⁶⁴Cu-DOTA-Benzene-HIB-Liposome was about 2%, which seemed numerically higher than the tumor uptake of mice 24 hours after injection of ⁶⁴Cu-DOTA-Benzene-HIB-Liposome, but the liposome circulates in vivo and, when the tumor uptake was confirmed, it was not high as compared to that in blood due to liposomes. The amount of uptake in the liver after 4 hours was significantly different from the amount of uptake in the liver after 24 hours, confirming that the tumor was rapidly escaping (FIG. 7).

### 4. ⁶⁴Cu-DOTA-Benzene-HIB-Liposome PET/CT

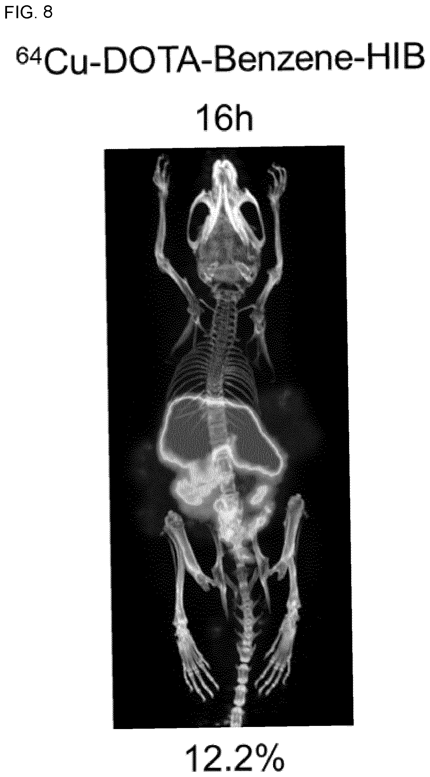

⁶⁴Cu-DOTA-Benzene-HIB was injected, and PET/CT analysis was performed 16 hours later. As a result of PET/CT analysis, the residual activity in vivo after 16 hours was 12.2%. A high uptake was shown in the liver, kidney, and intestines, such that typical *in vivo* excretion patterns were confirmed by imaging. As the signal from the heart was weak, it could be seen that the body was draining it well. The same *in vivo* excretion pattern as the result of tumor uptake was confirmed (FIG. 8).

### 5. Preparation of ¹⁷⁷Lu-DOTA-Benzene-HIB

Radiolabeling was initially performed using 0.25 M NH₄OAc buffer showing 76.51% radiolabeling. Conversion of the radiolabeling was monitored by radio-TLC iTLC (Bioscan 2000 imaging scanner).

Isotope Lu-177 was labeled on the synthesized DOTA-Benzene-HIB, and the labeling rate was confirmed by Radio-TLC, and the labeling rate was 76.51%.

### 6. Preparation of ¹⁷⁷Lu-DOTA-Benzene-HIB-Liposome

800 µL of 0.5mM DPPC, 350 µL of DSPE-PEG₂₀₀₀, and 50 µL of cholesterol were added to a 100 mL round flask. After lightly stirring them for about 5 seconds (using a chloroform solvent), a thin film was formed using an evaporator. After concentration under reduced pressure was completed, 1 mL of PBS was added to the round flask. Hydration was performed in a heating bath at 55°C. After hydration, the PBS solution was extracted with a glass syringe, and extrusion was performed 19 times each at 400 nm and 100 nm in size at 50 °C.

### 7. In vivo experiment of ¹⁷⁷Lu-DOTA-Benzene-HIB Liposome

### (1) Clearance

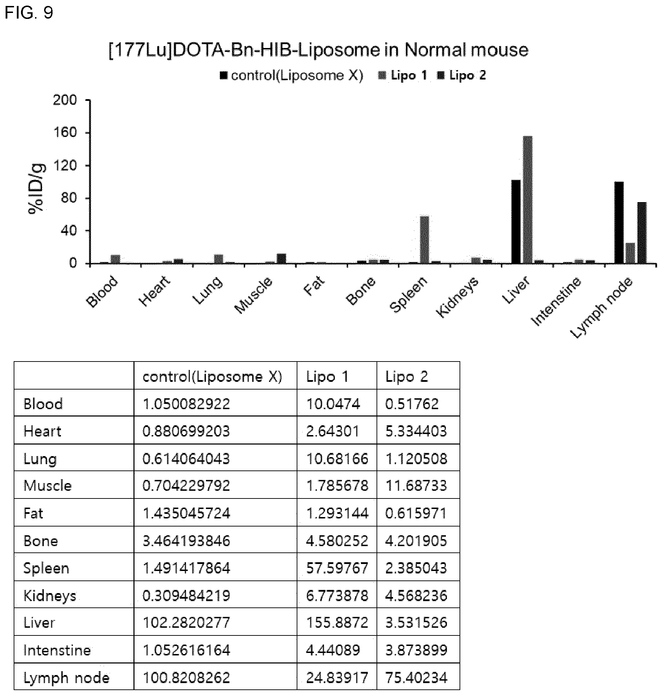

An experiment was conducted to determine the *in vivo* excretion rate of ¹⁷⁷Lu-DOTA-Benzene-HIB-Liposome. ⁶⁴Cu-DOTA-Benzene-HIB-Liposome was injected into mice and biodistribution was performed 24 hours later. As a result of biodistribution, uptake was confirmed in the blood and heart, confirming that liposome was *in vivo* circulating. It was confirmed that ⁶⁴Cu-DOTA-Benzene-HIB-Liposome had lower uptake in the liver, as compared to ⁶⁴Cu-DOTA-Benzene-HIB, and was excreted about 1.5 times faster (FIG. 9).

### (2) Tumor uptake

Biodistribution was performed to confirm the tumor uptake of ¹⁷⁷Lu-DOTA-Benzene-HIB-Liposome. ¹⁷⁷Lu-DOTA-Benzene-HIB-Liposome was injected and biodistribution was confirmed 24 hours later. As a result, it was confirmed that the tumor uptake of control and liposome was barely less than 1%. In addition, since uptake in the spleen was high, it could be expected that the liposome was broken (FIG. 10).

### III. Example 3: Synthesis of ATSM-mono-hexadecyl benzoate

### (1) Synthesis of hexadecyl 4-isothiocyanatobenzoate

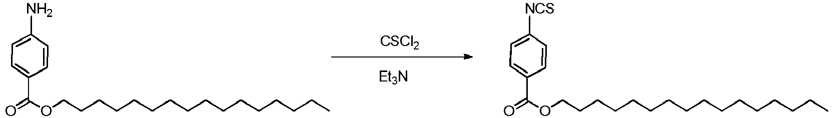

Hexadecyl 4-aminobenzoate (0.15 g, 1 eq) was dissolved in 50 ml of Ethyl acetate, and Et₃N (0.13 ml, 2.2 eq) were added to the solution at 0 °C. Then thiophosgene (0.056 g 1.1 eq) was added dropwise over 30 min under 0 °C. The reaction solution was stirred for 12 h at room temperature. Then, the solution was concentrated and extracted with EtOAc. Yellowish liquid (0.88 g, 93.7%). Used immediately for the next step without further purification.

### (2) Synthesis of ATSM-mono-hexadecyl benzoate

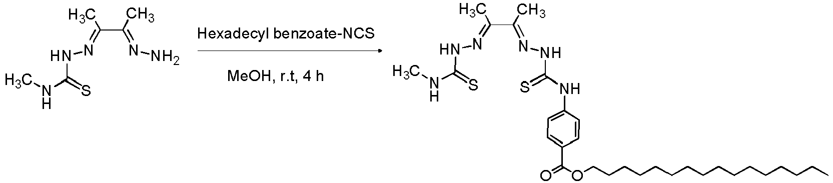

The ATSM intermediate (75 mg, 1 eq) compound was dissolved in methanol (15 mL) and added dropwise into the methanol solution of NCS-hexadecyl benzoate (150 mg, 1.01 mmol, 5 mL). The mixture was stirred at room temperature over the 4h. The yellow color precipitate was formed which was purified by column chromatography to afford final compound ATSM-mono-hexadecyl benzoae (69 mg, 29.2%). as a yellow solid.

### (3) Preparation of ⁶⁴Cu-ATSM-mono-hexadecyl benzoate

Complexation of ⁶⁴Cu with ATSM-HIB was performed by mixing 0.01 M HCl (1-5 µL) with a chelator (10 µg) solution in which carrier-free ⁶⁴CuCl₂ (18.5-111 MBq) was dissolved in ethanol (100 µL). Then, the reaction was carried out at 60 °C for 5-10 minutes in a thermomixer at 800 rpm. Conversion of radiolabeling was monitored by radio-TLC (Bioscan 2000 imaging scanner) [Silica TLC ethyl acetate:methanol=95:5], and the final radiochemical purity was ≥ 97%. Isotope Cu-64 was labeled on the synthesized ATSM-mono-hexadecyl benzoate, and the labeling rate was confirmed by Radio-TLC, and the labeling rate was 97.60%.

### IV. Example 4: Synthesis of ATSM-mono-hexadecyl benzene sulfonate

### (1) Synthesis of 4-isothiocyanatobenzne sulfonic acid

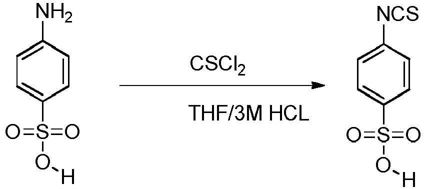

4-aminobenzoic sulfonic acid (3.0 g, 1 eq) was dissolved in 50 ml of THF were added to the solution at 0 °C. Then add thiophosgene (2.1 g, 1.1 eq) was added dropwise over 30 min under 0 °C. A 3 M HCl solution in which all starting materials were dissolved was added, subjected to reaction for 24 hours, and confirmed by Silica-TLC (ethyl acetate:hexane=9:1) to confirm a new spot. It was purified using a column to obtain a white solid (1.60 g, yield - 43%).

### (2) Synthesis of 4-aminobenzene-1-sulfonyl chloride

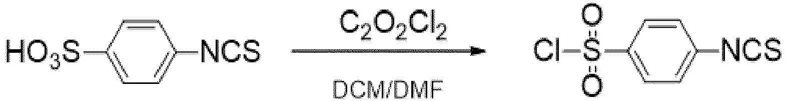

A solution of 4-isothiocyanatobenzne sulfonic acid (100 mg, 1eq) was dissolved in DCM (20 ml) and add CO₂Cl₂ (0.295 g, 5 eq) at 0 °C. Then add catalytic amount of DMF 10 µl. Stir the solution around 72 h at room temperature. Purified by column. Isolated-57.8 mg.

### (3) Synthesis of Hexadecyl 4-isothiocyanatobenzene sulfonate

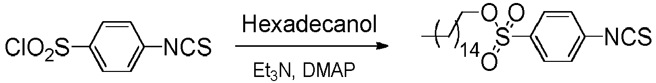

To a solution of 1-hexadecanol (600 mg, 1 eq), Et₃N (0.758 ml, 2 eq), DMAP (28.8 mg, 10 mol%) In DCM 10 ml. Add 4-NCS-Benzene sulfonyl chloride (640 mg, 1.1 eq) portion wise at 0 °C. The resulting mixture stirred at room temperature overnight. Purified by column using Hexane/EA (9/1). Isolated- 305 mg.

### (4) Synthesis of ATSM-mono-hexadecyl benzene sulfonate

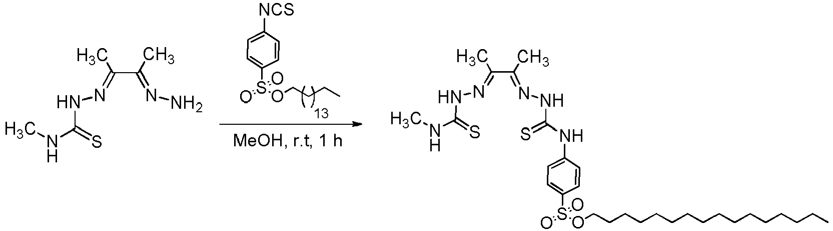

The ATSM intermediate (25 mg, 1 eq) was dissolved in methanol (15 mL) and added dropwise into the methanol solution of NCS-hexadecyl benzene sulfonate (64.5 mg, 1.01 mmol, 5 mL). The mixture was stirred at room temperature over the 24h. The yellow color precipitate was formed which was purified by column chromatography to afford final compound ATSM-mono-hexadecyl benzene sulfonate (28.2 mg) as a yellow solid.

### (5) ⁶⁴Cu-ATSM-mono-hexadecyl benzene sulfonate label

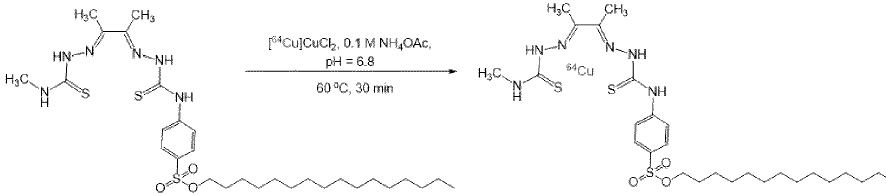

Complexation of the ⁶⁴Cu with ATSM-sulfate-HIB was performed by the mixing of no-carrier-added ⁶⁴CuCl₂ (18.5-111 MBq) in 0.01 M HCl (1-5 µL) with a solution of the chelator (10 µg) in ethanol (100 µL) followed by incubation at 60 °C for 5-10 min in a Thermomixer (800 rpm, Eppendorf). The conversion of the radiolabeling was monitored by radio-TLC (Bioscan 2000 imaging scanner) [Silica TLC Ethyl acetate:MeOH = 95:5] and the final radiochemical purity was ≥ 98%.

### V. Example 5: Synthesis of HIB-sulfate and its utilization

### 1. HIB-sulfate synthesis

### (1) Synthesis of cold-HIB-sulfonic acid

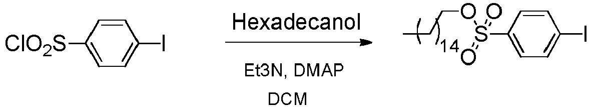

Add 4-lodo benzene sulfo chloride (0.935g, 1.5 eq) dissolved in CH₂Cl₂ dropwise to a solution of hexadecanol (0.5g, 1eq), Et₃N (0.57ml, 2eq), DMAP (25mg, 10% mol) in CH₂Cl₂ (5 mL). Stir the resulting mixture vigorously at room temperature 24h at 0 °C. Monitor the reaction by TLC. Treat the reaction mixture with saturated aqueous NaHCOs (10 mL). Stir the reaction mixture at room temperature for 20 minutes. Add ethyl acetate (20 mL) to the reaction mixture and separate the organic layer. Evaporate the solvent. Purify the crude product by chromatography on silica gel,

### (2) Synthesis of Stannylation of HIB-sulfonic acid

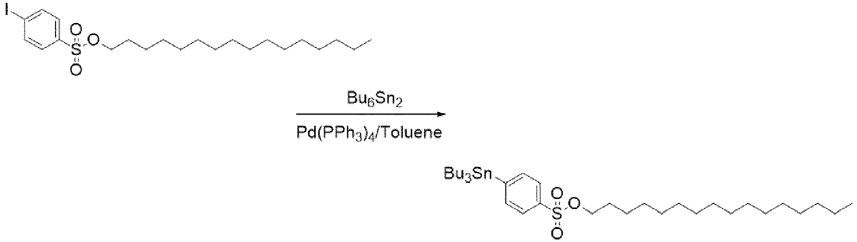

Add 4-lodo benzene hexadecyl sulfonate (0.1g, 1.0 eq) dissolved in toluene dropwise to a solution of Bu₆Sn₂ (0.125g, 1.1 eq), Pd(PPh₃)₄ at rt. Stir the resulting mixture vigorously at 100 °C for 24h. After completion of the reaction filter the solution and concentrate by rotary evaporator. Purify by column chromatography by using hexane and ethyl acetate (20:1).

### (3) ¹³¹I-HIB-sulfate label

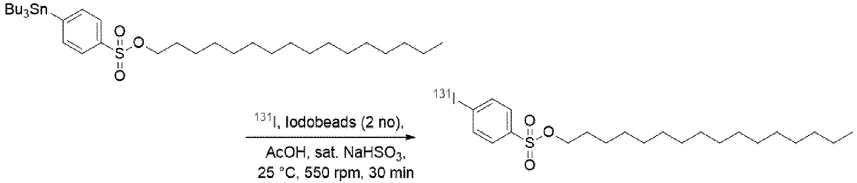

Stannylated HIB-sulphate precursor (100µg) dissolved in 100 µl AcOH was added in to the Na¹³¹I (500 µCi). Then the radioiodination was initiated by adding Iodobeads (2 balls) vortexed the reaction mixture for a few minutes and incubated the reaction at 25°C, 550 rpm, 30min. The progress of the reaction was monitored by Radio-TLC (Silica TLC hexane: ethyl acetate (20:1). Quench the reaction by using Na₂S₂O₅. Purification done by evaporation using rotary evaporator. The purified ¹³¹I-HIB-sulfate was isolated in 1 mL of CHCl₃ and proceeded further for liposome preparation.

Isotope I-¹³¹ was labeled on the synthesized HIB-sulfate, and the labeling rate was confirmed by Radio-TLC, and the labeling rate was 91.23%.

### (4) Preparation of ¹³¹I-HIB-sulfate-Liposome

800 µL of 0.5mM DPPC, 350 µL of DSPE-PEG₂₀₀₀, and 50 µL of cholesterol were added to a 100 mL round flask. After lightly stirring them for about 5 seconds (using a chloroform solvent), a thin film was formed using an evaporator. After concentration under reduced pressure was completed, 1 mL of PBS was added to the round flask. Hydration was performed in a heating bath at 55°C. After hydration, the PBS solution was extracted with a glass syringe, and extrusion was performed 19 times each at 400 nm and 100 nm in size at 50 °C.

### 2. In vivo experiment of ¹³¹I-HIB-sulfate-Liposome

### (1) tumor uptake (1)

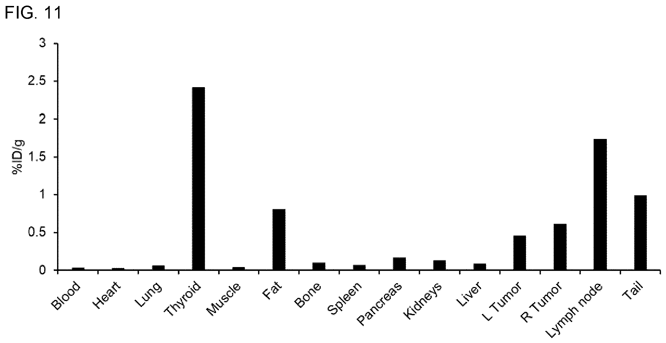

Biodistribution was performed to confirm the tumor uptake of ¹³¹I-HIB-sulfate-Liposome. There were CT26 tumors on both sides of the mouse, and ¹³¹I-HIB-sulfate-Liposome tumor uptake was injected and biodistribution was confirmed 24 hours later. As a result, after 24 hours, there was almost no *in vivo* residual Activity in the blood and heart, and it was confirmed that the *in vivo* excretion was well achieved. High uptake in the liver, kidneys, and organs confirmed a typical *in vivo* excretion pattern. Tumor uptake was as low as less than 1%. However, given the high uptake in Thyroid, it was expected that Free-I is present, which was expected to be caused by the liposome breaking and the labeled Iodine from HIB-sulfate being released (FIG. 11).

### (2) tumor uptake (2)

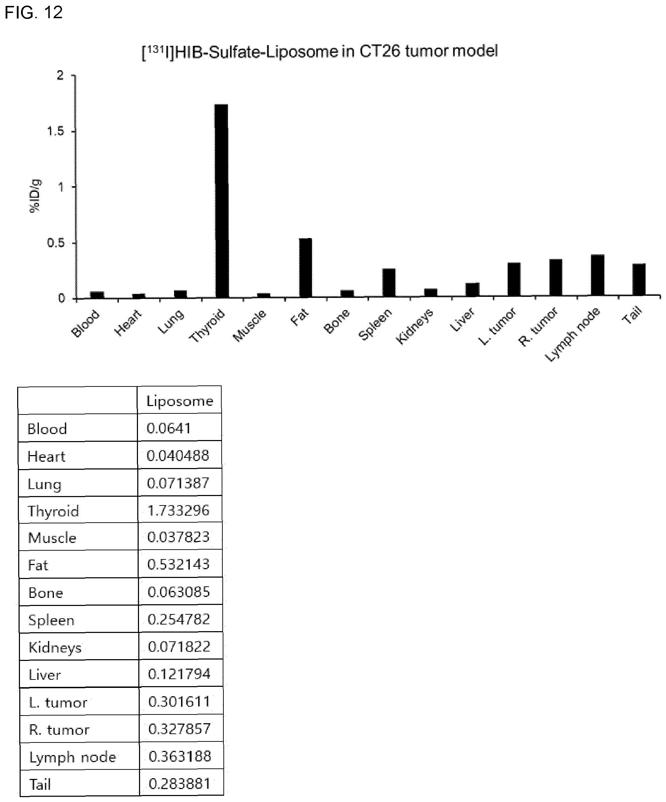

Biodistribution was performed to confirm the tumor uptake of ¹³¹I-HIB-sulfate-Liposome. There were CT26 tumors on both sides of the mouse, and ¹³¹I-HIB-sulfate-Liposome tumor uptake was injected and biodistribution was confirmed 24 hours later. As a result, after 24 hours, there was almost no *in vivo* residual Activity in the blood and heart, and it was confirmed that the *in vivo* excretion was well achieved. High uptake in the liver, kidneys, and organs confirmed a typical *in vivo* excretion pattern. Tumor uptake was as low as less than 1%. However, given the high uptake in Thyroid, it was expected that Free-I is present, which was expected to be caused by the liposome breaking and the labeled Iodine from HIB-sulfate being released (FIG. 12).

### (3) tumor uptake (3)

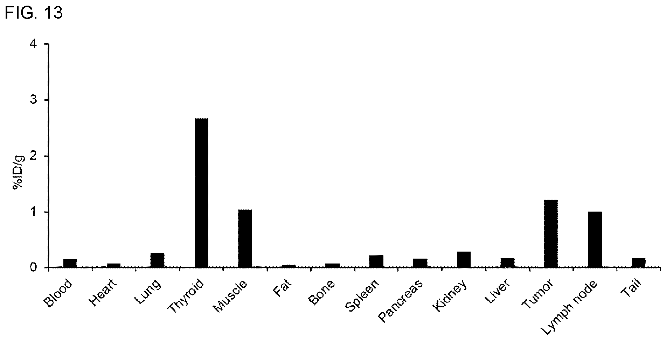

Biodistribution was performed to confirm the tumor uptake of ¹³¹I-HIB-sulfate-Liposome. The tumor model used is EMT6, and ¹³¹I-HIB-sulfate-Liposome tumor uptake was injected and biodistribution was confirmed 24 hours later. As a result, after 24 hours, there was almost no *in vivo* residual Activity in the blood and heart, and it was confirmed that the *in vivo* excretion was well achieved. High uptake in the liver, kidneys, and organs confirmed a typical *in vivo* excretion pattern. Tumor uptake was as low as less than 2%. However, given the high uptake in Thyroid, it was expected that Free-I was present, which was expected to be caused by the liposome breaking and the labeled Iodine from HIB-sulfate being released (FIG. 13).

### VI. Example 6: Synthesis of HIB-sulfate-amide

### (1) Synthesis of cold HIB-sulfonic acid amide

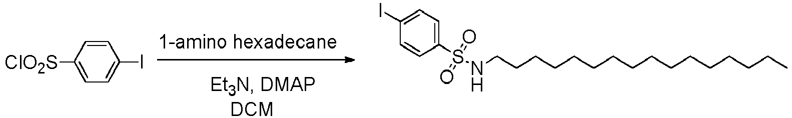

Add 4-lodo benzene sulfonyl chloride (1 g, 1.1 eq) dissolved in CH₂Cl₂ dropwise to a solution of 1-Amino hexadecane (0.75 g, 1eq), Et₃N (0.345 g, 1.1 eq) at 0 °C. Stir the resulted mixture vigorously at room temperature 24h. Monitor the reaction by TLC after completion of the reaction evaporate all solvent by rotary evaporator and purify the crude product by column chromatography on silica gel using hexane ethyl acetate (95:5) as an eluent.

### (2) Synthesis of Stannylation of HIB-sulfonic acid amide

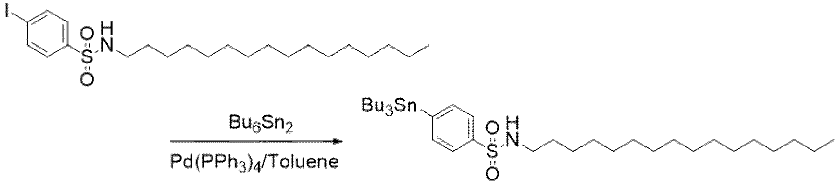

Add 4-lodo benzene hexadecyl sulfonyl amide (0.1 g, 1.0 eq) dissolved in toluene dropwise to a solution of Bu₆Sn₂ (0.125 g, 1.1 eq), Pd(PPh₃)₄ at rt. Stir the resulting mixture vigorously at 100 °C for 24h. After completion of the reaction filter the solution and concentrate by rotary evaporator. Purify by column chromatography by using hexane and ethyl acetate (20:1).

### (3) ¹³¹I-HIB-sulfate-amid label

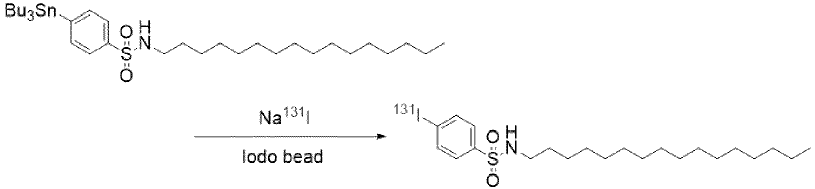

Stannylated HIB-sulphate amide precursor (100µg) dissolved in 100 µl AcOH and then added into the Na¹³¹I (500 µCi). Then the radioiodination was initiated by adding Iodobeads (2 balls) vortexed the reaction mixture for a few minutes and incubate the reaction at 25°C, 550 rpm, 30min. The progress of the reaction was monitored by Radio-TLC (Silica TLC hexane: ethyl acetate (20:1). Quench the reaction by using Na₂S₂O₅.

Isotope I-¹³¹ was labeled on the synthesized HIB-sulfate (2), and the labeling rate was confirmed by Radio-TLC, and the labeling rate was 85.59%.

### VII. Example 7: Synthesis of nido-Carborane-HIB and its utilization

### 1. Synthesis of nido-Carborane-HIB

### (1) Synthesis of Carborane-HIB

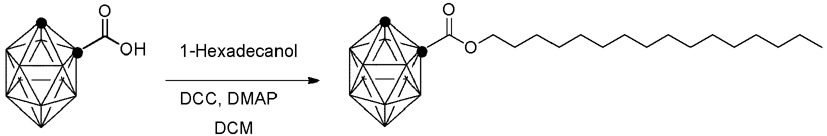

A solution of carborane carboxylic acid (0.1 g, 1eq), N,Ndicyclohexylcarbodiimide (0.13 g, 1.2 eq), hexadecyl alcohol (0.14g, 1.2) and 4-dimethyhuninopyridine (0.060 g 1.2 eq) in dry dichloromethane (25 ml) was stirred at room temperature under argon for 56 hrs. Purification was performed after the reaction.

### (2) Synthesis of nido-carborane-HIB

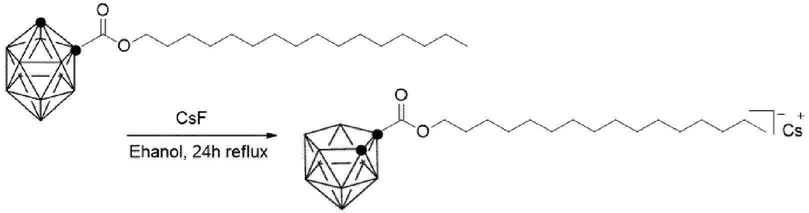

A mixture of o-carborane-HIB (30 mg, 1eq) and cesium fluoride (63 mg, 2 eq) in ethanol (10 mL) was stirred under reflux conditions for 24 h. The clear solution was dried by a rotary evaporator to give a white solid that was dissolved in 10 mL of acetone. The solution was filtered off by glass frit to remove the white borate and washed several times with the same solvent. The white product obtained by the removal of the solvent of the filtrate in vacuo.

### (3) ¹³¹I-nido-Carborane-HIB label

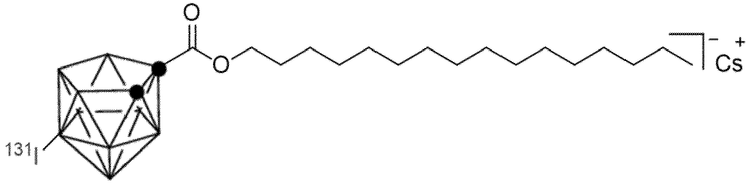

A solution of *nido*-Carborane-HIB(100 µg/10 µl DI water) was added into the Na¹³¹I solution (540 µCi) then added 5% AcOH in H₂O (100 µl). The reaction was initiated by adding freshly prepared chloramine-T (5 µl; 5 mg/ml) solution to vortex the reaction mixture and incubate for 30 min at room temperature. Completion of the reaction was monitor by radio TLC: C18, ACN: H₂O (1:1).

Isotope I-¹³¹ was labeled on the synthesized nido-Carborane-HIB, and the labeling rate was confirmed by Radio-TLC, and the labeling rate was 98.21%.

### 2. In vivo experiment of ¹³¹I-nido-Carborane-HIB

### (1) clearance

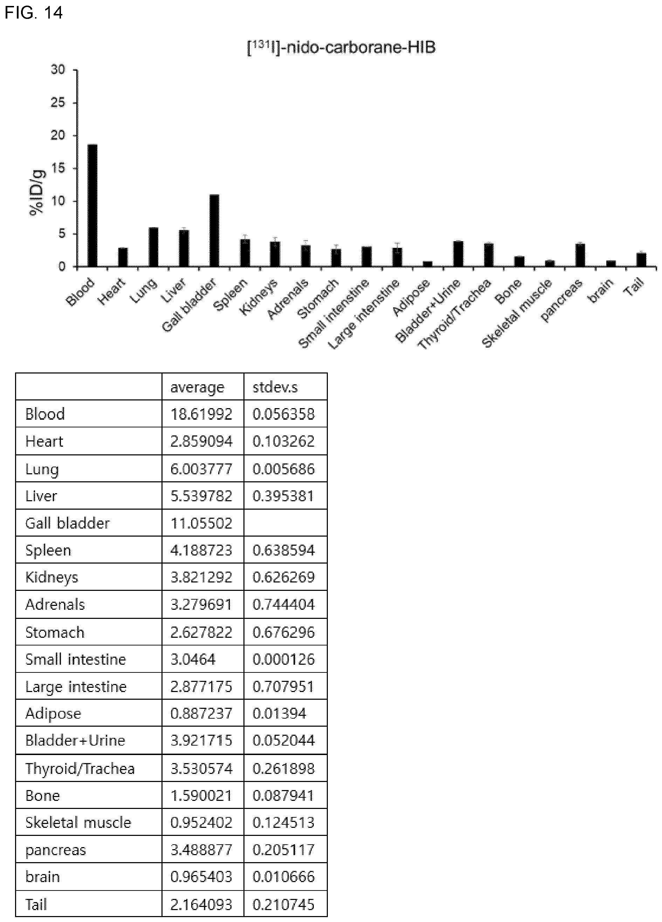

An experiment was conducted to determine the *in vivo* excretion rate of ¹³¹I-nido-Carborane-HIB. ¹³¹I-*nido*-Carborane-HIB was injected into mice and biodistribution was performed 24 hours later. As a result of biodistribution, it was confirmed that the *in vivo* residual Activity was 67%, and excretion was not performed well. Since the uptake in blood was high, it was confirmed that the liposome was *in vivo* circulating. As a result of checking the uptake patterns in various organs, high uptake was confirmed in the gallbladder. In addition, it was confirmed that uptake in the brain was low, and it did not pass the Blood-Brain Barrier. Since the uptake in the bladder and urine was high, it was confirmed that Activity was leaking through urine (FIG. 14).

### (2) PET/CT imaging

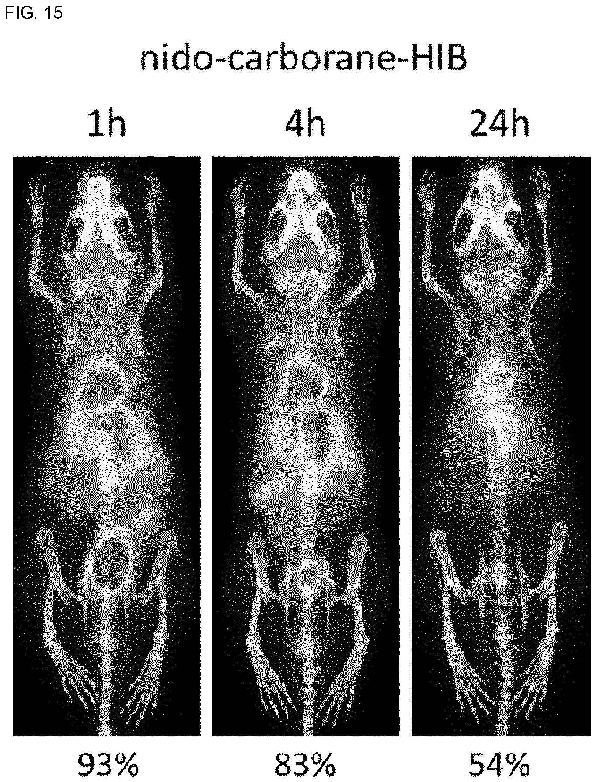

I-124 was labeled using the same labeling method as I-131, and the *in vivo* excretion process was confirmed through PET/CT analysis. It was confirmed that the *in vivo* residual activity was 93% after 1 hour, 83% after 4 hours, and 54% after 24 hours, steadily decreasing over time. As the liver, kidney, and intestine showed high uptake, the typical *in vivo* excretion pattern could be confirmed through images, and the *in vivo* excretion rate was confirmed to be slow. It was confirmed that most of the *in vivo* Activity was excreted through urine. However, since the signal from the heart was strong, it was confirmed that *in vivo* circulation was becoming active (FIG. 15).

### VIII. Example 8: Synthesis of C-OH-nido-Carborane-HIB

### (1) Synthesis of C-OH-carborane-HIB

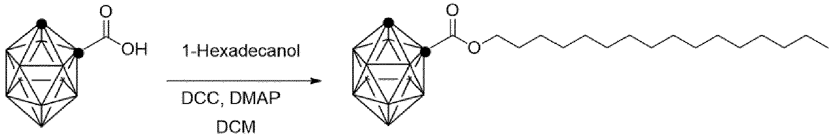

A solution of C-OH-carborane carboxylic acid (25 g, 1eq), N, N dicyclohexylcarbodiimide (13 mg), hexadecyl alcohol (14 mg) and 4-dimethylaminopyridine (6 mg) in dry DCM (5 ml) was stirred at room temperature. Purification was done by column chromatography using hexane and ethyl acetate(20:1) under argon for 56 hrs.

### (2) Synthesis of C-OH-nido-carborane-HIB

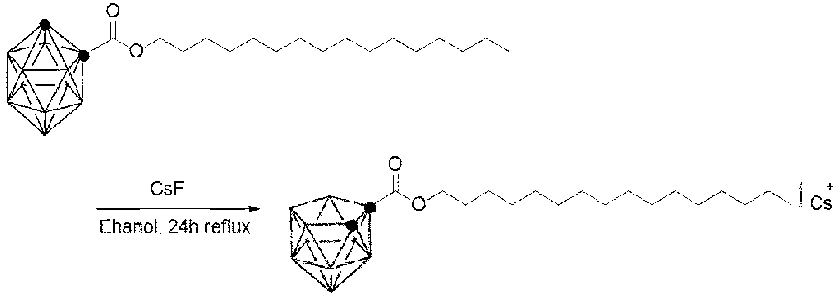

A mixture of C-OH *nido*-carborane-HIB (30 mg, 1eq) and cesium fluoride (63 mg, 2 eq) in ethanol (10 mL) was stirred under reflux conditions for 24 h. The clear solution was dried by a rotary evaporator to give a white solid that was dissolved in 10 mL of acetone. The solution was filtered off by glass frit to remove the white borate and washed several times with the same solvent. The white product obtained by the removal of the solvent of the filtrate in vacuo.

### (3) ¹³¹I-C-OH-nido-carborane-HIB label

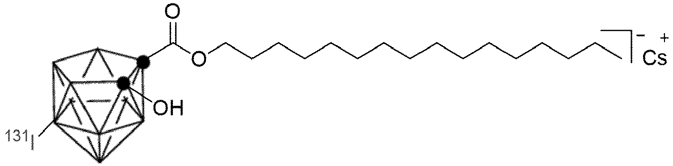

A solution of C-OH *nido*-Carborane-HIB(100 µg/10 µl DI water) was added in to the Na¹³¹I solution (540 µCi) then add 5% AcOH in H₂O (100 µl). The reaction was initiated by adding freshly prepared chloramine-T (5 µl; 5 mg/ml) solution to vortex the reaction mixture and incubate for 30 min at room temperature. Completion of the reaction was monitor by radio TLC: C18, ACN: H₂O (1:1).

### INDUSTRIAL APPLICABILITY

A novel compound of chemical Formulas 1 to 8 according to the present invention exhibits selectivity according to the difference in activity of esterase for each organ due to ester residue in a structure, and thus is extremely effective in visualizing pancreatic cancer, etc. Therefore, the present invention has a high industrial applicability.

## Claims

1. A novel radioactive compound selected from the group consisting of Formulas 1 to 8 or a pharmaceutically acceptable salt thereof: 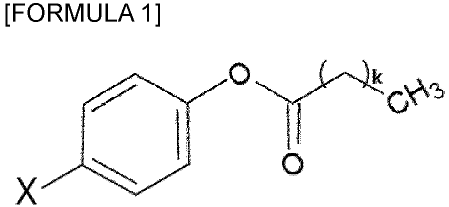 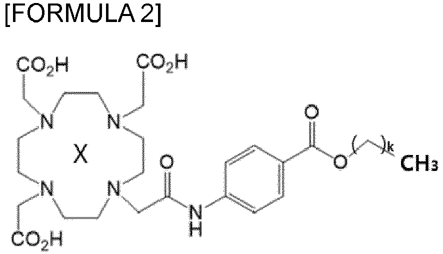 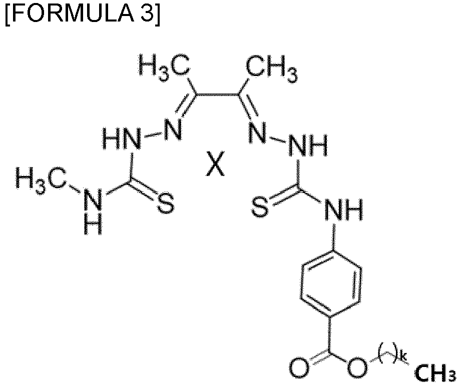 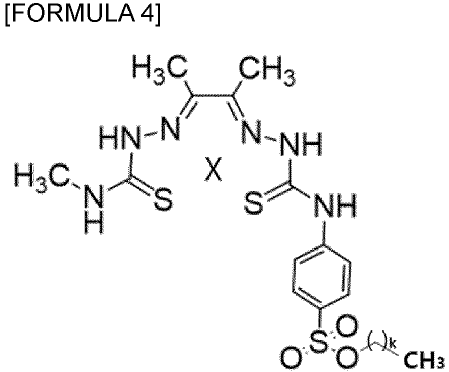 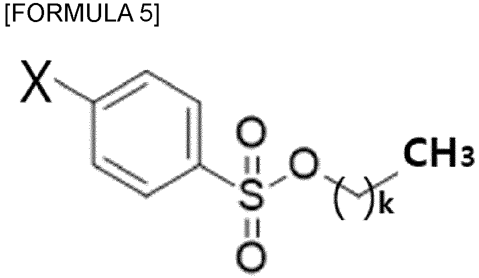 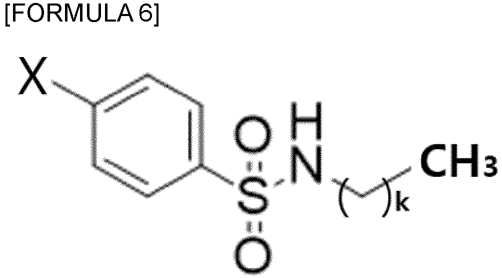 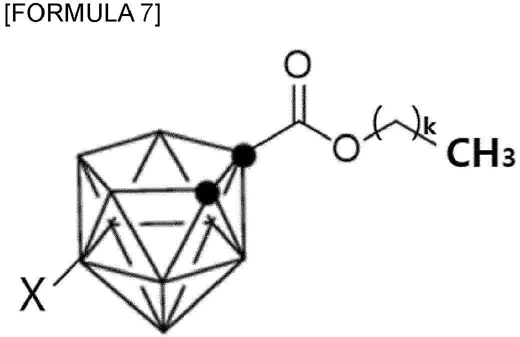 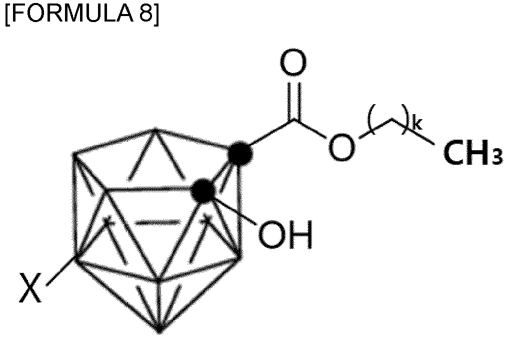
wherein, in the Formulas 1 to 8, X is a diagnostically active radioisotope selected from the group consisting of ⁴³Sc, ⁴⁴Sc, ⁵¹Mn, ⁵²Mn, ⁶⁴Cu, ⁶⁷Ga, ⁶⁸Ga, ⁸⁶Y, ⁸⁹Zr, ^{94m}Tc, ^{99m}Tc, ¹¹¹In, ¹⁵²Tb, ¹⁵⁵Tb, ²⁰¹ Tl, ²⁰³Pb, ¹⁸F, ⁷⁶Br, ⁷⁷Br, ¹²³I, ¹²⁴I and ¹²⁵I; or a therapeutically active radioisotope selected from the group consisting of ⁴⁷Sc, ⁶⁷Cu, ⁸⁹Sr, ⁹⁰Y, ¹⁵³Sm, ¹⁴⁹Tb, ¹⁶¹Tb, ¹⁷⁷Lu, ¹⁸⁶Re, ¹⁸⁸Re, ²¹²Pb, ²¹³Bi, ²²³Ra, ²²⁵Ac, ²²⁶Th, ²²⁷Th, ¹³¹I and ²¹¹At,
k is an integer from 5 to 30.

2. A liposome comprising the compound of claim 1 or a pharmaceutically acceptable salt thereof; and lipids.

3. The liposome of claim 2, wherein the lipids comprise (a) cholesterol; (b) 1,2-dipalmitoyl-sn-glycero-3-phosphocholine (DPPC); and (c) 1,2-distearoyl-*sn*-glycero-3-phosphoethanolamine-*N*-[methoxy(polyethylene glycol)-2000(DSPE-PEG₂₀₀₀).

4. The liposome of claim 2, wherein a portion of the total DSPE-PEG₂₀₀₀ comprised in the liposome is attached to folic acid.

5. A composition for *in vivo* imaging comprising the compound of claim 1 or a pharmaceutically acceptable salt thereof; or the liposome of claim 2.

6. The composition of claim 5, wherein the imaging is for computed tomography (CT), magnetic resonance imaging (MRI), positron emission tomography (PET) and single-photon emission computed tomography (SPECT).

7. A composition for diagnosing a cancer comprising the compound of claim 1 or a pharmaceutically acceptable salt thereof; or the liposome of claim 2.

8. The composition of claim 7, wherein the cancer is pancreatic cancer.

9. A composition for preventing or treating a cancer comprising the compound of claim 1 or a pharmaceutically acceptable salt thereof; or the liposome of claim 2.

10. A compound of formula 9 or 10, or a pharmaceutically acceptable salt: 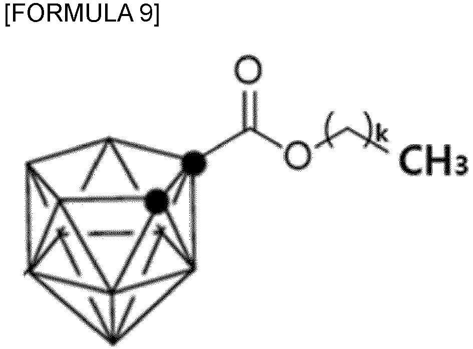 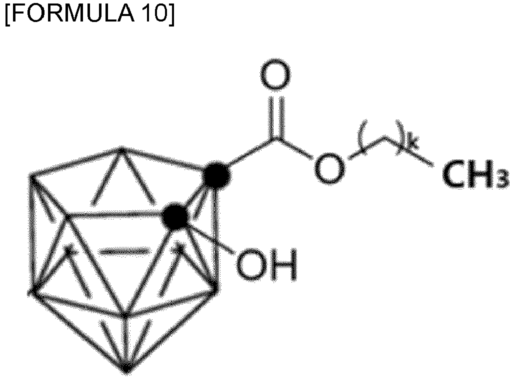
wherein, in the formula 9 or 10,
k is an integer from 5 to 30.

11. Use of the compound of claim 1 or a pharmaceutically acceptable salt thereof, or the liposome of claim 2 for preparing a composition for *in vivo* imaging.

12. A method for *in vivo* imaging comprising the steps of:
i) administering the compound of claim 1 or a pharmaceutically acceptable salt thereof, or the liposome of claim 2 to a subject;
ii) waiting for a sufficient time to allow the compound or liposome to be introduced into the subject's body; and
iii) imaging an area where the compound or liposome is detected in the subject.

13. Use of the compound of claim 1 or a pharmaceutically acceptable salt thereof, or the liposome of claim 2 for preparing a composition for diagnosing a cancer.

14. A method for diagnosing a cancer comprising the steps of:
a) administering the compound of claim 1 or a pharmaceutically acceptable salt thereof, or the liposome of claim 2 to a subject;
b) waiting for a sufficient time to allow the compound or liposome to be introduced into cancer cells;
c) detecting proliferative cells by imaging an area where the compound or liposome is detected in the subject; and
d) diagnosing the subject as having cancer when the compound or liposome is detected in the subject.

15. Use of the compound of claim 1 or a pharmaceutically acceptable salt thereof, or the liposome of claim 2 for preparing a composition for preventing or treating a cancer.

16. A method for treating a cancer, the method comprising administering an effective amount of a composition comprising the compound of claim 1 or a pharmaceutically acceptable salt thereof, or the liposome of claim 2 to a subject in need thereof.
